# EUROPEAN PATENT APPLICATION

(11) **EP 4 721 733 A1**
(43) Date of publication of application: **08.04.2026**
(21) Application number: 23939275.6
(22) Date of filing: 07.11.2023
(51) Int. Cl.: A61K 9/51, A61K 47/26, A61K 9/19

(54) **NUCLEIC ACID-LIPID NANOPARTICLE COMPOSITION, AND LYOPHILIZED PREPARATION THEREOF, PREPARATION METHOD THEREFOR AND USE THEREOF**

(30) Priority: 02.06.2023 WO PCT/CN2023/098109
(71) Applicant: Cansino Biologics Inc., Tianjin 300457 (CN); Cansino (Shanghai) Biological Research Co., Ltd., Shanghai 201203 (CN)
(72) Inventor: GE, Chen, Tianjin 300457 (CN); ZHU, Tao, Tianjin 300457 (CN); QIU, Dongxu, Tianjin 300457 (CN); LIU, Jian, Tianjin 300457 (CN); WANG, Haomeng, Tianjin 300457 (CN); HE, Jun, Tianjin 300457 (CN); YAN, Zhihong, Tianjin 300457 (CN); WANG, Zhenghua, Tianjin 300457 (CN)
(74) Representative: Finnegan Europe LLP
(86) International application number: PCT/CN2023/130039
(87) International publication number: WO 2024/244304

(57) **Abstract**

The present disclosure relates to a nucleic acid-lipid nanoparticle composition and a lyophilized preparation thereof, and further relates to a preparation method for the composition and a use of the composition. The nucleic acid-lipid nanoparticle composition of the present disclosure comprises lactate, so that the stability of nucleic acid-lipid nanoparticles is improved in a lyophilization process and a prepared lyophilized preparation. According to the present disclosure, the total time of a freeze-drying process for the nucleic acid-lipid nanoparticles can be shortened, and energy consumption and the time cost of product scale-up production can be greatly reduced; the rehydration of lyophilized nucleic acid-lipid nanoparticles is quick, and the total amount of nucleic acids, the encapsulation efficiency, and the nucleic acid integrity are high; in addition, a lyophilized and rehydrated preparation has cell transfection efficiency not significantly different from that of a non-lyophilized nucleic acid-lipid nanoparticle stock solution, and has a high in vivo immune response, even exceeding that of the non-lyophilized nucleic acid-lipid nanoparticle stock solution.

## Description

### TECHNICAL FIELD

The present disclosure relates to the technical field of biopharmaceuticals, and particularly, to a nucleic acid-lipid nanoparticle composition, a lyophilized formulation of the composition, and a method for preparing same and use thereof.

### BACKGROUND

In the COVID-19 epidemic, mRNA vaccines were approved for emergency use in the US and EU due to the advantages of safety, high immune response, and cost-efficiency. In recent years, studies have found that transient protein expression induced by mRNA has great application values in various fields such as vaccines for other infectious diseases, cancer vaccines, cardiovascular diseases, protein replacement therapy, genetic diseases, and the like, and even can achieve the autonomous CAR-T effect *in vivo.* However, unlike most vaccines stored at 2-8 °C, approved mRNA vaccines have poor long-term stability, require cryopreservation (at -20 °C, or even -70 °C), and have a shelf life of less than 6 months. Stability becomes the primary limitation in use when hundreds of millions of mRNA vaccine doses need to be stored, transported, and distributed around the world.

The length of the mRNA chain is 1000 to 5000 bases, and earlier studies have found that naked mRNA can be rapidly hydrolyzed by ribonuclease (RNase). RNase is a ubiquitous enzyme present both *in vivo* and *in vitro,* and can only be removed by special treatment. In addition, the integrity of the mRNA molecules in mRNA vaccines is critical since a simple change in the long chain of mRNA (breakage or base oxidation) may terminate the translation.

The expression of target proteins from mRNA in organisms requires intact mRNA molecules. Therefore, the integrity of the mRNA molecules in mRNA vaccines is critical since a simple change in the long chain of mRNA (breakage or base oxidation) may result in the failure in translation. Meanwhile, a study by Fabre et al. showed that in the same conditions, the degradation rate in the integrity of mRNA molecules was positively correlated with the length of the mRNA molecules, and a longer mRNA molecule may be more readily prone to degradation in the integrity during storage. The lengths of two approved mRNA vaccines against SARS-CoV-2 are about 4000 bases, while the length of self-amplifying mRNA products that are in clinical studies may even be 10,000 or more bases. To reduce the degradation rate in integrity of mRNA molecules is becoming more urgent.

The lyophilization of mRNA formulations is an effective means for improving the stability of the mRNA formulations, and several studies have been conducted on the lyophilization of mRNA formulations. In 2009, a patent entitled "LYOPHILIZATION OF NUCLEIC ACIDS IN LACTATE-CONTAINING SOLUTIONS" invented by MUTZKE, TM et al. of CureVac about mRNA lyophilization discloses a process for preparing a lyophilized formulation from a protamine-mRNA complex proprietary to CureVac. In the disclosure, an 80% lactated Ringer's solution is preferred as a solvent for preparing the mRNA solution, and the prepared mRNA solution and protamine are mixed in a ratio of 4:1 (w/w). The mRNA-protamine mixture is then lyophilized, and the lyophilized mRNA formulation has better integrity after standing at room temperature. The rationale of the disclosure is that the presence of the 80% lactated Ringer's solution may influence the complexing of protamine and mRNA to form a stable structure, thus possessing good stability at room temperature after lyophilization.

The lactated Ringer's solution, as a solvent with a high content of salts, is generally considered unsuitable for the lyophilization of lipid nanoparticles in the prior art, since nucleic acid-lipid nanoparticles having a high salt content will be damaged after lyophilization. Thus, the use of the lactated Ringer's solution by CureVac as a solvent for lyophilization cannot be replicated in nucleic acid-lipid nanoparticle formulations.

In 2022, Muramatsu et al. (in cooperation with BioNTech corporation) reported a lyophilized mRNA formulation with good stability in long-term storage. The lyophilized mRNA-LNP formulation has small quality attribute changes after standing at 4 °C for 24 weeks with no significant decrease in *in-vivo* bioactivity, and thus has significantly improved stability compared with a frozen liquid formulation. After the lyophilized formulation was stood at 25 °C or 42 °C, the integrity and the *in-vivo* activity both exhibited a certain descending trend, and in the article, after the lyophilized formulations of two shorter mRNA sequences Luc mRNA and PR8 HA mRNA were stood at 42 °C for one month, the integrity was sharply reduced from the initial integrity about 100% to 30-40%, and correspondingly, the titer of the mouse immune antibody was 1-2 orders of magnitude lower than that of the initial titer. This indicates that even in the case of lyophilized formulations, a significant reduction in integrity may occur in a short term when stored at room temperature or above, which is closely related to the reduction in *in-vivo* activity.

To improve the stability of mRNA formulations, in the prior art, the mRNA formulations are usually prepared into lyophilized formulations to reduce the occurrence of hydrolysis. However, even after the mRNA formulation is prepared into a lyophilized formulation, at least 1-2% of water contained in the final formulation is difficult to completely remove, and the water still causes mRNA hydrolysis.

Therefore, there is an urgent need for new lyophilized formulations that better avoid mRNA hydrolysis than those in the prior art, thus giving mRNA formulations of higher stability.

### SUMMARY

The first objective of the present disclosure is to improve the storage stability of nucleic acid-lipid nanoparticles by reducing the storage conditions to refrigeration (at 2-8 °C) or room temperature storage (at 10-30 °C), extending the shelf life, and providing a fast reconstitution in water. The shelf life may be 18 months or longer in refrigeration conditions, and 12 months or longer at room temperature.

The second objective of the present disclosure is to significantly reduce the overall duration of the lyophilization process, increase efficiency, and improve the energy-efficiency and efficiency production.

The third objective of the present disclosure is to find out the specific identity and amount of the lyoprotectant for the nucleic acid-lipid nanoparticles, so as to maintain the total nucleic acid amount, encapsulation efficiency, and integrity of the formulation after lyophilization and reconstitution with no loss in *in-vivo* and *in-vitro* biological activity.

The fourth objective of the present disclosure is to break through the empirical development mode of lyophilization processes and establish a method for assessing critical physicochemical attributes.

Although it is generally recognized in the prior art that the introduction of salts is disadvantageous to the lyophilization of lipid nano-particle formulations, the inventors surprisingly found the addition of a lactate salt, such as sodium lactate, to a nucleic acid-lipid nanoparticle formulation improves the stability of the nucleic acid-lipid nanoparticles after lyophilization. On this basis, the present disclosure is completed.

In one aspect, the present disclosure provides a nucleic acid-lipid nanoparticle composition, comprising: a nucleic acid, a lipid, and a lactate, wherein the lactate is selected from sodium lactate or potassium lactate in an amount of 5-100 mM, for example, 5-75 mM, 5-50 mM, or 10-100 mM, preferably 5-50 mM.

In some embodiments, the nucleic acid-lipid nanoparticle composition further comprises a buffer, wherein the content ratio of the buffer to the lactate is (0.25-1):1.

In some embodiments, the buffer is one selected from or a combination of more selected from the group consisting of an acetate buffer, a PBS buffer, a citrate buffer, a phosphate buffer, a histidine buffer, and a tris buffer. The content of the buffer is 1.25-100 mM, for example, 2.5-100 mM, 5-100 mM, 5-75 mM, or 5-50 mM; preferably, the content of the buffer is 1.25-50 mM or 5-50 nM.

In some embodiments, the nucleic acid-lipid nanoparticle composition further comprises a lyoprotectant.

In some embodiments, the lyoprotectant is one selected from or a combination of more selected from the group consisting of sucrose, trehalose, lactose, cyclodextrin, and mannitol.

In some embodiments, the pH of the nucleic acid-lipid nanoparticle composition is 1.0-14.0, preferably 5.0-9.0, and more preferably 7.0-8.0.

In some embodiments, the nucleic acid is selected from the group consisting of ssDNA, dsDNA, mRNA, lncRNA, siRNA, saRNA, shRNA, ASO, plasmid, circRNA, circDNA, miRNA, CRISPR-Cas, ployI:C, SamRNA, and 5'-pppRNA.

In some embodiments, the nucleic acid is encapsulated in a lipid nanoparticle (LNP).

In some embodiments, the lipid comprises a cationic lipid, a neutral phospholipid, a PEG-lipid, and optionally a steroidal lipid.

In some embodiments, the composition is further prepared into a lyophilized formulation.

In some embodiments, the content of the lactate is 0.5-10.0 wt%, for example, 0.5-7.5 wt%, 0.5-5.0 wt%, or 1.0-10 wt%, preferably 0.5-5.0 wt%, on dry basis by the weight of the lyophilized formulation.

In some embodiments, the content of the buffer is 0.35-10.0 wt%, for example, 0.35-7.5 wt%, 0.35-5.0 wt%, or 1.0-10 wt%, preferably 0.5-5.0 wt%, on dry basis by the weight of the lyophilized formulation.

In another aspect, the present disclosure provides a method for preparing a nucleic acid-lipid nanoparticle composition, wherein the composition is prepared into a lyophilized formulation, and the method comprises:
(1) mixing a lactate solution with a nucleic acid-lipid nanoparticle stock solution, or
   adding a lactate to a nucleic acid solution before encapsulating the nucleic acid, and adding a lipid, or
   ultrafiltering a nucleic acid-lipid nanoparticle into a solution containing lactate,
   wherein the lactate is selected from sodium lactate or potassium lactate in an amount of 5-100 mM, for example, 5-75 mM, 5-50 mM, or 10-100 mM, preferably 5-50 mM;
(2) adding a lyoprotectant; and
(3) lyophilizing to give a lyophilized formulation of the nucleic acid-lipid nanoparticle composition.

In some embodiments, the lyoprotectant is one selected from or a combination of more selected from the group consisting of sucrose, trehalose, lactose, cyclodextrin, and mannitol.

In some embodiments, the content of the lyoprotectant is 50-99.9 wt%, on dry basis by the weight of the lyophilized formulation.

In some embodiments, step (1) further comprises adding a buffer, the content ratio of the buffer to the lactate being (0.25-1):1.

In some embodiments, the buffer is one selected from or a combination of more selected from the group consisting of an acetate buffer, a PBS buffer, a citrate buffer, a phosphate buffer, a histidine buffer, and a tris buffer. The content of the buffer is 1.25-100 mM, for example, 2.5-100 mM, 5-100 mM, 5-75 mM, or 5-50 mM; preferably, the content of the buffer is 1.25-50 mM or 5-50 nM.

In some embodiments, the pH of the solution prepared in step (1) is 1.0-14.0, for example, 2.0-13.0, preferably 5.0-9.0, and more preferably 7.0-8.0.

In some embodiments, the nucleic acid is selected from the group consisting of ssDNA, dsDNA, mRNA, lncRNA, siRNA, saRNA, shRNA, ASO, plasmid, circRNA, circDNA, miRNA, CRISPR-Cas, ployI:C, SamRNA, and 5'-pppRNA.

In some embodiments, in step (1), the nucleic acid is encapsulated in a lipid nanoparticle.

In some embodiments, the lipid comprises a cationic lipid, a neutral phospholipid, a PEG-lipid, and optionally a steroidal lipid.

In still another aspect, the present disclosure provides use of the nucleic acid-lipid nanoparticle composition disclosed herein in preparing a medicament.

In some embodiments, the medicament is a vaccine.

In some embodiments, the vaccine is a therapeutic tumor vaccine or a vaccine for preventing a cancer, a viral infection, a bacterial infection, or a fungal infection.

In some embodiments, the virus is selected from the group consisting of norovirus, Ebola virus, coronavirus, cytomegalovirus, Dengue virus, Zika virus, enterovirus, hepatitis virus, herpes simplex virus, human papilloma virus, influenza virus, Marburg virus, measles virus, poliovirus, rabies virus, and rotavirus.

In some embodiments, the medicament is a formulation for oral administration, intramuscular injection, subcutaneous injection, intravenous injection, or inhalation.

In some embodiments, the formulation for inhalation is an atomized inhalant or a dry powder inhalant.

Compared with the prior art, the present disclosure has the following beneficial effects:
In order to solve the mRNA hydrolysis problem due to the residual water in the existing lyophilized formulations, an additive capable of reducing the activity of water molecules is introduced to reduce the mRNA molecule hydrolysis in lyophilized formulations. Lactates, such as sodium lactate, as natural, safe and non-toxic substances, are utilized to further improve the stability of the mRNA molecules in the lyophilized formulations due to their effects of reducing the activity of water molecules.

The present disclosure overcomes the recognition in the prior art that the introduction of salts can adversely effect the lyophilization of lipid nanoparticle formulations. In the present disclosure, the lactate, such as sodium lactate, is separately added for the lyophilization of the nucleic acid-lipid nanoparticle to improve the stability of the nucleic acid-lipid nanoparticle after lyophilization. According to the present disclosure, the lactate and the buffer are compounded to further improve the stability of the nucleic acid-lipid nanoparticle after lyophilization. Compared with the lyophilized formulations reported in the prior art, the nucleic acid-lipid nanoparticle after lyophilization prepared by the present disclosure possesses significantly improved stability, and exhibits a high level of integrity and good *in-vivo* activity after exposure to a high temperature of 42 °C. The present disclosure can improve the storage temperature and shelf life of the lyophilized nucleic acid-lipid nanoparticle formulation and significantly reduce the storage and transportation costs.

The nucleic acid-lipid nanoparticle of the present disclosure has good lyophilization properties, with no significant changes in quality attributes of the lyophilized formulation such as appearance, reconstitution time, residual water, particle size, potential, encapsulation efficiency, mRNA content and integrity compared with those before lyophilization, and comparable cell transfection efficiency and *in-vivo* activity to those before lyophilization.

The inventors found that the stability of the lyophilized nucleic acid-lipid nanoparticle formulation is closely correlated with the pH of the nucleic acid-lipid nanoparticle before lyophilization, and it was also found that the pH of the nucleic acid-lipid nanoparticle before lyophilization should be properly maintained at 2-13, preferably, 7-8.

Compared with the lyophilized mRNA-LNP formulations reported in the prior art, the addition of the lactate significantly improves the stability of the nucleic acid-lipid nanoparticle after lyophilization prepared by the present disclosure, which exhibits a high level of integrity and good *in-vivo* activity after exposure to a high temperature of 42 °C. The present disclosure can improve the storage temperature and shelf life of the lyophilized mRNA-LNP formulation and significantly reduce the storage and transportation costs.

Although the introduction of salts is considered to be very unfavorable for the lyophilization of nano-formulations such as LNP in the prior art, the present disclosure, by exploring the proper amount and the mode of addition of lactates into the lyophilized mRNA-LNP formulation, still produces good lyophilized products by using mRNA-LNP containing lactates. The lyophilized formulation exhibits no significant changes in quality attributes such as appearance, reconstitution time, residual water, particle size, potential, encapsulation efficiency, mRNA content and integrity compared with those before lyophilization, and comparable cell transfection efficiency and *in-vivo* activity to those before lyophilization.

The method can reduce the overall duration of the lyophilization process of the nucleic acid-lipid nanoparticles to 8 to 18 hours, and significantly improve the energy-efficiency and efficiency of scale-up production. The lyophilized nucleic acid-lipid nanoparticle prepared by the lyophilization process disclosed herein features a fast reconstitution in water (in 10 s) and a high total nucleic acid amount, encapsulation efficiency and nucleic acid integrity. In addition, the formulation after lyophilization and reconstitution demonstrates a cell transfection efficiency with no significant difference from that of the original solution of the non-lyophilized nucleic acid-lipid nanoparticle, and a high immune response *in vivo* even superior to that of the original solution of the non-lyophilized nucleic acid-lipid nanoparticle. In addition, by reducing and even eliminating the water-containing microenvironment in the system through lyophilization, the nucleic acid hydrolysis reaction catalyzed by the nuclease can be suppressed, thus significantly improving the long-term stability of the nucleic acid-lipid nanoparticle, prolonging the shelf life, increasing the storage temperature to 2-8 °C, and significantly improving the cost-efficiency and efficiency of storage and transportation. In addition, changing the phase of the nucleic acid-lipid nanoparticle formulation from liquid to solid has important significance to the development of novel solid formulations.

### BRIEF DESCRIPTION OF THE DRAWINGS

In order to more clearly illustrate the specific embodiments of the present disclosure and embodiments in the prior art, the drawings used for the specific embodiments and the prior art are briefly introduced below. It is obvious that the drawings in the following description are only some embodiments of the present disclosure, and other drawings can be obtained by those of ordinary skills in the art without creative efforts.
FIG. 1 illustrates the cryogenic electron microscopy results of the mRNA-LNP formulation after lyophilization and reconstitution in Example 1-1.
FIG. 2a illustrates the integrity determination profile after lyophilization and reconstitution in Example 2-1.
FIG. 2b illustrates the integrity determination profile after lyophilization and reconstitution in Example 2-2.
FIG. 2c illustrates the integrity determination profile after lyophilization and reconstitution in Example 2-3.
FIG. 3 illustrates the integrity changes in three groups of lyophilized formulations in Example 2-1, Example 2-2, and Example 2-3 after exposure to a temperature of 42 °C.
FIG. 4a illustrates the integrity determination profile after lyophilization and reconstitution in Example 2-4.
FIG. 4b illustrates the integrity determination profile after lyophilization and reconstitution in Example 2-5.
FIG. 4c illustrates the integrity determination profile after lyophilization and reconstitution in Example 2-6.
FIG. 5 illustrates the gE protein expression in cells after mRNA-LNP transfection.
FIG. 6 illustrates the cellular immune response (Elispot) in mice after two immunizations.
FIG. 7 illustrates the humoral immune response in mice on day 14 after the primary immunization and day 14 after the secondary immunization.
FIG. 8 illustrates the expression in cells transfected with mRNA carrying different eGFP sequences before and after lyophilization.
FIG. 9 illustrates the cryogenic electron microscopy results of the mRNA-LNP formulation after lyophilization and reconstitution in Example 6-1.
FIG. 10 illustrates the cryogenic electron microscopy results of the mRNA-LNP formulation after lyophilization and reconstitution in Example 6-2.
FIG. 11 illustrates the expression in cells transfected with different protonatable cationic lipid-encapsulated eGFP mRNA sequences before and after lyophilization.
FIG. 12 illustrates the cryogenic electron microscopy results of the mRNA-LNP formulation after lyophilization and reconstitution in Example 7-1.
FIG. 13 illustrates the cryogenic electron microscopy results of the mRNA-LNP formulation after lyophilization and reconstitution in Example 7-2.
FIG. 14 illustrates the cryogenic electron microscopy results of the mRNA-LNP formulation after lyophilization and reconstitution in Example 7-3.

### DETAILED DESCRIPTION

The foregoing and other objectives, components, and advantages will be apparent from the following detailed descriptions of specific embodiments, as illustrated and exemplified in the accompanying drawings, wherein like reference numerals, identifiers, and the like refer to like elements, components, or features of the specific embodiments. It is noted that the processes described below, unless otherwise specified, are all those that can be achieved or understood by those skilled in the art with reference to the prior art. Reagents or instruments without specified manufacturers used herein are conventional products that are commercially available.

For the extent of use, the terms "comprising," "including," and "having," as used in the CLAIMS and SPECIFICATION, or any variations thereof, are intended to be inclusive of the open group of other elements not specified. The terms "at least one" and "one or more" can be used interchangeably. The term "single" is used to indicate that one and only one object is intended. Similarly, when a specific number of objects are required, other particular integer values, such as "two," will be used. The terms "preferably," "preferable," "preferred," "optionally," "may," and similar terms are used to indicate that an item, a condition, or a procedure being referred to is an optional (i.e., not necessary) feature of the embodiments. Ranges described as "a to b" include the values "a" and "b" unless otherwise indicated.

Although various modifications have been described herein with reference to specific embodiments of the present disclosure, it should be appreciated that such descriptions are made only by way of illustration and should not be construed as limiting the scope of the present disclosure. Accordingly, the scope and content of the present disclosure are only defined by the appended claims, in their current form or form as amended during examination or as amended during prosecution. Furthermore, it should be appreciated that, unless otherwise indicated, features of any specific embodiment discussed herein may be combined with one or more features of any one or more embodiments otherwise discussed or contemplated herein.

### Definitions

As used herein, the term "nucleic acid" described herein refers to a polymer containing at least two deoxyribonucleotides or ribonucleotides in either single- or double-stranded form and includes DNA, RNA, and hybrids or derivatives thereof, e.g., double-stranded RNA, single-stranded RNA, isolated RNA (e.g., partially purified RNA), substantially pure RNA, synthetic RNA, recombinantly produced RNA, and altered RNA that differs from a natural RNA by the addition, deletion, substitution, and/or alteration of one or more nucleotides. Such alterations may include, for example, the addition of non-nucleotide species to the end or interior (e.g., at one or more nucleotides of the RNA) of an interfering RNA. The nucleotides in the RNA molecules of the present disclosure may also include non-standard nucleotides, such as non-natural nucleotides, chemically synthesized nucleotides, or deoxynucleotides. Such altered RNAs can be referred to as analogs or analogs of natural RNA. As used herein, the terms "ribonucleic acid" and "RNA" refer to molecules containing at least one ribonucleotide residue, including siRNA, antisense RNA, single-stranded RNA, microRNA, mRNA, non-coding RNA, and multivalent RNA. Ribonucleotides are nucleotides with a hydroxyl group at position 2' of the β-D-ribofuranose moiety. Such terms include double-stranded RNA (dsRNA), single-stranded RNA (ssRNA), isolated RNA (e.g., partially purified RNA), substantially pure RNA, synthetic RNA, recombinantly produced RNA, and modified and altered RNA that differs from a natural RNA by the addition, deletion, substitution, modification, and/or alteration of one or more nucleotides. The alteration of RNA may include, for example, the addition of non-nucleotide species to the end or interior (e.g., at one or more nucleotides of the RNA) of an interfering RNA. The nucleotides in the RNA molecules may also include non-standard nucleotides, such as non-natural nucleotides, chemically synthesized nucleotides, or deoxynucleotides. Such altered RNAs may be referred to as RNA analogs.

As used herein, the term "lipid nanoparticle" refers to a particle having at least one nanoscale dimension comprising at least one lipid, which is a lipid preparation useful for delivering a therapeutic nucleic acid (e.g., mRNA) to a target site of interest (e.g., a cell, tissue, an organ). In some embodiments, the lipid nanoparticle is a nucleic acid-lipid particle, which is generally composed of a nucleic acid, a cationic lipid, a non-cationic lipid (e.g., a phospholipid), an associated polymer lipid (e.g., a PEG-lipid) that prevents the aggregation of the particle, and optionally a structural lipid (e.g., cholesterol). In general, the therapeutic nucleic acid (e.g., mRNA) may be encapsulated in the lipid moiety of the lipid nanoparticle, thereby protecting it from enzymatic degradation, which may also be referred to as a "nucleic acid-lipid nanoparticle". As used herein, the term "lipid" refers to a class of organic compounds that are derivatives (e.g., esters) of fatty acids and are generally characterized by insolubility in water but solubility in many organic solvents. Lipids generally fall into at least three categories: (1) "simple lipids" including fats, oils, and waxes; (2) "complex lipids" including phospholipids and glycolipids; and (3) "derived lipids" such as steroids.

As used herein, the terms "cationic lipid" and "ionizable lipid" are used interchangeably herein to include those lipids having one, two, three, or more fatty acid or fatty alkyl chains and a pH titratable amino head group (e.g., an alkylamino or dialkylamino head group), and salts thereof. Cationic lipids are typically protonated (i.e., positively charged) at a pH below the pKa of the cationic lipid and are substantially neutral at a pH above the pKa. The cationic lipids of the present disclosure may also be referred to as titratable cationic lipids.

As used herein, the term "phospholipid" refers to a lipid molecule consisting of two hydrophobic fatty acid "tails" and a hydrophilic "head" consisting of a phosphate group. The two components are most often held together by a glycerol molecule, and therefore, in the present disclosure, the phospholipid is preferably a neutral phospholipid. In addition, the phosphate group is often modified by simple organic molecules such as choline (i.e., to give phosphorylcholine) or ethanolamine (i.e., to give phosphoethanolamine). In some embodiments, the phospholipid may be selected from the group including, but not limited to: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-di-undecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-diarachidonoyl-sn-glycero-3-phosphoethanolamine, 1,2-dididocosahexaenoyl-sn-glycero-3-phosphoethanolamine, 1,2-dioleoyl-sn-glycero-3-phosphate-rac-(1-glycerol) sodium salt (DOPG), sphingomyelin, and a mixture thereof.

As used herein, the term "polymeric lipid" primarily includes PEG lipids, also known as PEGylated lipids or PEG-lipids (polyethylene glycol lipids), referring to any suitable lipids modified with a PEG (polyethylene glycol) group. In some embodiments, the PEG lipid may be selected from the group including, but not limited to, a PEG-modified phosphatidylethanolamine, a PEG-modified phosphatidic acid, a PEG-modified ceramide, a PEG-modified dialkylamine, a PEG-modified diacylglycerol, a PEG-modified dialkylglycerol, and a mixture thereof. In some embodiments, specific examples of PEG lipids include, but are not limited to, C14-PEG2000 (1,2-dimyristoyl-rac-glycerol, methoxypolyethylene glycol-2000 (DMG-PEG2000)) and C18-PEG5000 (1,2-distearoyl-rac-glycerol, methoxypolyethylene glycol-5000 (DSG-PEG5000)).

As used herein, the term "steroidal lipid," which may also be referred to as a "structural lipid," refers to a steroidal compound (also referred to as a sterol or steroid in some cases) or a lipid containing a steroidal moiety.

As used herein, the term "lyoprotectant" refers to one or more compounds that increase the chemical and/or physical stability of a lipid compound or composition, e.g., a lipid nanoparticle, during the lyophilization, storage, or reconstitution of the lipid compound or composition. In some embodiments, the lyoprotectant is selected from a saccharide, a polyol, or a derivative thereof. In some embodiments, the lyoprotectant is selected from a monosaccharide, a disaccharide, or a mixture thereof. For example, the lyoprotectant may be sucrose, lactulose, trehalose, lactose, glucose, maltose, mannitol, cellobiose, cyclodextrin, or a mixture thereof. It should be noted that the lyoprotectant used in the present disclosure may be a lyoprotectant well-known by those skilled in the art and commonly used, and is not specified in the present disclosure.

The term "vaccine" used herein refers to a composition suitable for application to an animal (including a human), which induces an immune response after administration with a strength sufficient to help prevent, improve, or cure clinical diseases caused by microbial infections as a minimum.

The term "delivery system" used herein refers to a formulation or composition that regulates the spatial, temporal, and dose distribution of a biologically active ingredient in an organism.

### Nucleic Acid-Lipid Nanoparticle

The nucleic acid is encapsulated in a lipid moiety of the lipid nanoparticle to give the nucleic acid-lipid nanoparticle.

In some embodiments, the nucleic acid includes, but is not limited to, ssDNA, dsDNA, mRNA, lncRNA, siRNA, saRNA, shRNA, ASO, plasmid, circRNA, circDNA, miRNA, CRISPR-Cas, ployI:C, SamRNA, or 5'-pppRNA. In some specific embodiments, the nucleic acid is mRNA. In some embodiments, the lipid comprises a cationic lipid, a neutral phospholipid, a PEG-lipid, and optionally a steroidal lipid. For example, in some embodiments, the lipid comprises a cationic lipid, a neutral phospholipid, and a PEG-lipid. In some embodiments, the lipid comprises a cationic lipid, a neutral phospholipid, a PEG-lipid, and a steroidal lipid.

In some embodiments, the cationic lipid comprises an ionizable tertiary amine (e.g., pH titratable) head group; C18 alkyl chains, wherein each alkyl chain independently has 0 to 3 (e.g., 0, 1, 2, or 3) double bonds; and ether, ester or ketal bonds between the head group and the alkyl chains. Such cationic lipids include, but are not limited to, DSDMA, DODMA, DLinDMA, DLenDMA, γ-DLenDMA, DLin-K-DMA, DLin-K-C2-DMA (also referred to as DLin-C2K-DMA, XTC2, or C2K), DLin-K-C3-DMA, DLin-K-C4-DMA, DLen-C2K-DMA, y-DLen-C2K-DMA, DLin-MC2-DMA (also referred to as MC2), DLin-MC3-DMA (also referred to as MC3), DLin-MP-DMA (also referred to as 1-B11), and ALC-0315.

In some embodiments, the cationic lipid has the following structure: 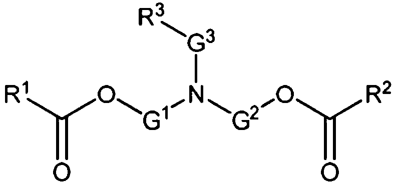 or a pharmaceutically acceptable salt or stereoisomer thereof, wherein:
G¹ and G² are each independently unsubstituted C₆-C₁₀ alkylene;
G³ is unsubstituted C₁-C₁₂ alkylene;
R¹ and R² are each independently C₆-C₂₄ alkyl or C₆-C₂₄ alkenyl;
R³ is OR⁵, CN, -C(=O)OR⁴, -OC(=O)R⁴, or -NR⁵C(=O)R⁴;
R⁴ is C₁-C₁₂ hydrocarbyl; and
R⁵ is H or C₁-C₆ hydrocarbyl.

In some specific embodiments, the cationic lipid is a compound having the following structure: 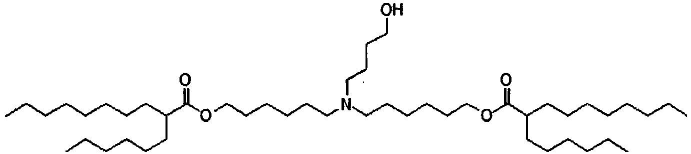 or, 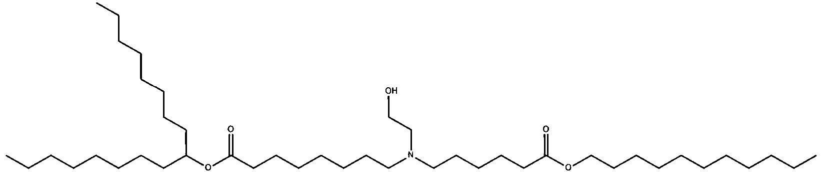 or, 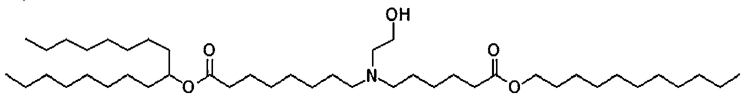 or, 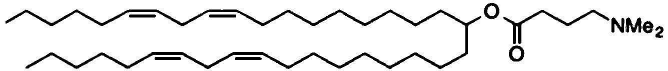 or, 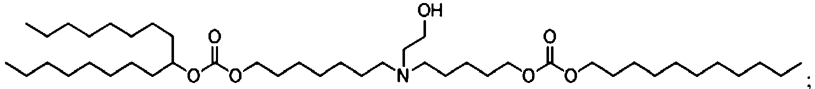 or, 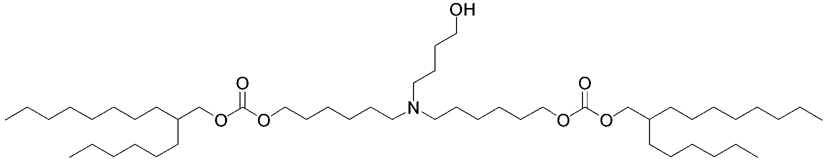 or, 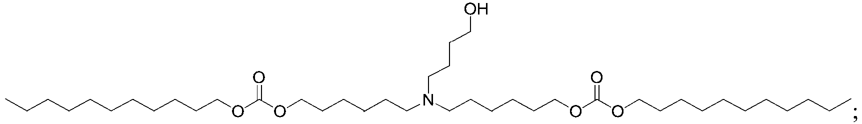 or, 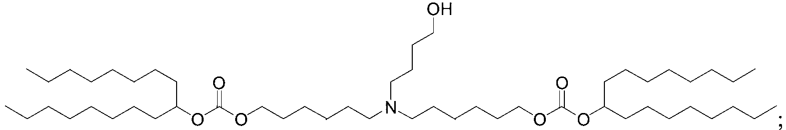 or, 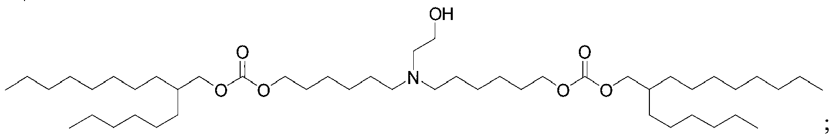 or,

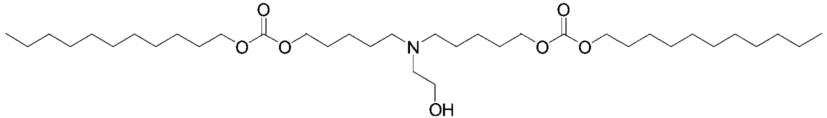

In some specific embodiments, the cationic lipid is a compound having the following structure:
DLin-MC3-DMA 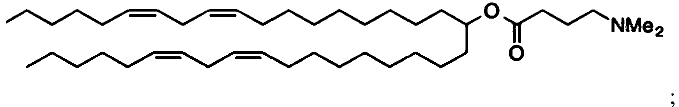
ALC-0315 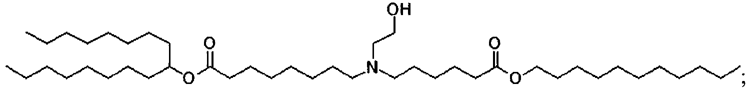
SM-102

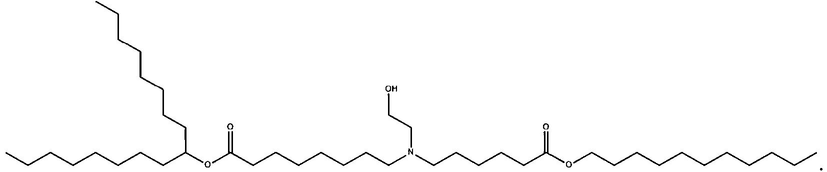

In some embodiments, the neutral phospholipid (which may also be referred to as and used interchangeably with a neutral lipid in the context of the present disclosure) is one selected from or a combination of more selected from the group consisting of 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), 1-palmitoyl-2-oleoyl phosphatidylethanolamine (POPE). In some specific embodiments, the neutral phospholipid is DSPC.

In some embodiments, the PEG-lipid is one selected from or a combination of more selected from the group consisting of 2-[(polyethylene glycol)-2000]-N,N-ditetradecylacetamide (ALC-0159), 1,2-dimyristoyl-sn-glycero-methoxypolyethylene glycol (PEG-DMG), 1,2-distearyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE), PEG-1,2-dimyristoylacyloxypropyl-3-amine (PEG-c-DMA) and DMG-PEG2000. In some specific embodiments, the PEG-lipid is DMG-PEG2000.

In some embodiments, the steroidal lipid is one selected from or a combination of more selected from the group consisting of avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, dinosterol, epicholesterol, ergosterol, fucosterol, hexahydrosterol, hydroxycholesterol and a polypeptide-modified cholesterol, lanosterol, photosterol, fucasterol, sitostanol, sitosterol, stigmastanol, stigmasterol, cholic acid, glycocholic acid, taurocholic acid, deoxycholic acid, and lithocholic acid. In some specific embodiments, the steroidal lipid is cholesterol.

In some specific embodiments, the lipid comprises a cationic lipid such as DLin-MC3-DMA, ALC-0315 or SM-102, a PEG-lipid such as DMG-PEG2000, a neutral phospholipid such as DSPC, and optionally a steroidal lipid such as cholesterol.

In some embodiments, the lipid nanoparticle comprises 25-75% of the cationic lipid, 5-20% of the neutral phospholipid, 1-5% of the PEG-lipid, and 0-50% of steroidal lipid, in molar percentage.

In some embodiments, in the lipid nanoparticle, the lipid component comprises 20-60% of the cationic lipid, 5-25% of the neutral phospholipid, 25-55% of the steroidal lipid, and 0.1-15% of the PEG-lipid, in molar percentage.

In some embodiments, in the lipid nanoparticle, the molar ratio of cationic lipid:neutral phospholipid:PEG-lipid:steroidal lipid is (30-60):(1-20):(0.1-10):(20-50). In some embodiments, in the lipid nanoparticle, the molar ratio of cationic lipid:neutral phospholipid:PEG-lipid:steroidal lipid is (40-60):(10-20):(1-5):(30-50). In some embodiments, in the lipid nanoparticle, the molar ratio of cationic lipid:neutral phospholipid:PEG-lipid:steroidal lipid is 45:10:2:43 or 40:10:2:48.

In the nucleic acid-lipid nanoparticle, the nucleic acid is encapsulated in the lipid nanoparticle formed of the lipid. In some embodiments, the mass ratio (w/w) of the nucleic acid to total lipid in the lipid nanoparticle is in the range of 1:(10-30).

In some embodiments, the nucleic acid-lipid nanoparticle has an average particle size of 50-200 nm. In some embodiments, the nucleic acid-lipid nanoparticle has a net neutral charge at a neutral pH. In some embodiments, the nucleic acid-lipid nanoparticle has a polydispersity index of less than 0.4.

The method for preparing the nucleic acid-lipid nanoparticle of the present disclosure comprises encapsulating the nucleic acid in the lipid nanoparticle, specifically, dissolving the cationic lipid, the neutral phospholipid, the PEG-lipid, and optionally the steroidal lipid into a solvent, and then mixing with the nucleic acid.

In some embodiments, the method for preparing the nucleic acid-lipid nanoparticle comprises dissolving the cationic lipid, the neutral phospholipid, the PEG-lipid, and optionally the steroidal lipid in ethanol, mixing with a diluted mRNA solution, ultrafiltering, diluting, and filtering, preferably, dissolving the cationic lipid, the neutral phospholipid, the PEG-lipid and the optional steroid lipid in ethanol, mixing with a diluted mRNA solution according to a certain flow rate ratio, ultrafiltering, diluting, and filtering, wherein preferably, the ultrafiltration is a tangential flow filtration, and more preferably, the mixing may be a turbulent mixing, a laminar mixing, or a microfluidic mixing.

### Nucleic Acid-Lipid Nanoparticle Composition

The present disclosure provides a nucleic acid-lipid nanoparticle composition, comprising: a nucleic acid, a lipid, and a lactate, wherein the lactate is selected from sodium lactate or potassium lactate in an amount of 5-100 mM, for example, 5-75 mM, 5-50 mM, or 10-100 mM, preferably 5-50 mM. The nucleic acid, the lipid, and the nucleic acid-lipid nanoparticle are as defined herein.

In some embodiments, the nucleic acid-lipid nanoparticle composition further comprises a buffer, wherein the content ratio of the buffer to the lactate is (0.25-1):1.

In some embodiments, the buffer is one selected from or a combination of more selected from the group consisting of an acetate buffer, a PBS buffer, a citrate buffer, a phosphate buffer, a histidine buffer, and a tris buffer. The content of the buffer is 1.25-100 mM, for example, 2.5-100 mM, 5-100 mM, 5-75 mM, or 5-50 mM; preferably, the content of the buffer is 1.25-50 mM or 5-50 nM.

In some embodiments, the nucleic acid-lipid nanoparticle composition further comprises an additional excipient that is one selected from or a combination of more selected from the group consisting of sodium acetate, tromethamine, potassium dihydrogen phosphate, sodium chloride, disodium hydrogen phosphate, and sucrose.

In some embodiments, the pH of the nucleic acid-lipid nanoparticle composition is 1.0-14.0, for example, 1.0, 2.0, 3.0, 4.0, 5.0, 6.0, 7.0, 8.0, 9.0, 10.0, 11.0, 12.0, 13.0, 14.0, and a range with any two of the above values as endpoints and a value in the range, for example, 2.0-13.0, 3.0-12.0, 4.0-11.0, 5.0-10.0, 5.0-9.0, 6.0-9.0, 6.0-8.0, 6.0-7.0, and 7.0-8.0. In some embodiments, the pH of the nucleic acid-lipid nanoparticle composition is 7.0, 7.1, 7.2, 7.3, 7.4, 7.5, 7.6, 7.7, 7.8, 7.9, 8.0, or a range with any two of the above values as endpoints and a value in the range. In some embodiments, the nucleic acid is selected from the group consisting of ssDNA, dsDNA, mRNA, lncRNA, siRNA, saRNA, shRNA, ASO, plasmid, circRNA, circDNA, miRNA, CRISPR-Cas, ployI:C, SamRNA, and 5'-pppRNA.

In some embodiments, the lipid comprises a cationic lipid, a neutral phospholipid, a PEG-lipid, and optionally a steroidal lipid.

In some embodiments, the nucleic acid is encapsulated in a lipid nanoparticle (LNP).

In some embodiments, the nucleic acid-lipid nanoparticle composition further comprises a lyoprotectant, and is further prepared into a lyophilized formulation. Non-limiting examples of the lyoprotectant are one selected from or a combination of more selected from the group consisting of sucrose, trehalose, lactose, cyclodextrin, and mannitol.

In a non-limiting embodiment, the lyoprotectant comprises sucrose and trehalose in a mass ratio of (5-15):(5-11). In a non-limiting embodiment, the lyoprotectant comprises one or more of sucrose, trehalose, mannitol, glucose, or lactose. In a non-limiting embodiment, the lyoprotectant comprises one or more of sucrose, trehalose, and one of mannitol, glucose or lactose in a mass ratio of (5-15):(5-11):(1-5). In a non-limiting embodiment, the lyoprotectant is prepared by dissolving the components in nuclease-free water in the corresponding ratio.

### Lyophilized Formulation of Nucleic Acid-Lipid Nanoparticle Composition

The present disclosure provides a lyophilized formulation of a nucleic acid-lipid nanoparticle composition. The nucleic acid-lipid nanoparticle composition is defined herein.

In some embodiments, the nucleic acid-lipid nanoparticle composition further comprises a lyoprotectant. The lyoprotectant is defined herein.

In some embodiments, the composition is further prepared into a lyophilized formulation.

In some embodiments, the content of the lactate is 0.5-10.0 wt%, for example, 0.5-7.5 wt%, 0.5-5.0 wt%, or 1.0-10 wt%, preferably 0.5-5.0 wt%, for another example, 0.5 wt%, 1.0 wt%, 1.5 wt%, 2.0 wt%, 2.5 wt%, 3.0 wt%, 3.5 wt%, 4.0 wt%, 4.5 wt%, 5.0 wt%, 5.5 wt%, 6.5 wt%, 7.0 wt%, 7.5 wt%, 8.0 wt%, 8.5 wt%, 9.0 wt%, 9.5 wt%, 10.0 wt%, or a range with any two of the values in the above ranges as endpoints, on dry basis by the weight of the lyophilized formulation.

In some embodiments, the content of the buffer is 0.35-10 wt%, for example, 0.35-7.5 wt%, 0.35-5.0 wt%, or 1.0-10 wt%, preferably 0.5-5.0 wt%, for another example, 0.35 wt%, 0.5 wt%, 0.75 wt%, 1.0 wt%, 1.5 wt%, 2.0 wt%, 2.5 wt%, 3.0 wt%, 3.5 wt%, 4.0 wt%, 4.5 wt%, 5.0 wt%, 5.5 wt%, 6.5 wt%, 7.0 wt%, 7.5 wt%, 8.0 wt%, 8.5 wt%, 9.0 wt%, 9.5 wt%, 10.0 wt%, or a range with any two of the values in the above ranges as endpoints, on dry basis by the weight of the lyophilized formulation.

In some embodiments, the content of the lyoprotectant is 50-99.9 wt%, for example, 50%, 55%, 60%, 65%, 70%, 75%, 80%, 85%, 90%, or 95%, for example, a range with any two of the values in the above ranges as endpoints, on dry basis by the weight of the lyophilized formulation.

### Method for Preparing Nucleic Acid-Lipid Nanoparticle Composition

The present disclosure provides a method for preparing a nucleic acid-lipid nanoparticle composition, wherein the composition is prepared into a lyophilized formulation, and the method comprises:
(1) mixing a lactate solution with a nucleic acid-lipid nanoparticle stock solution, or
   adding a lactate to a nucleic acid solution before encapsulating the nucleic acid, and adding a lipid, or
   ultrafiltering a nucleic acid-lipid nanoparticle into a solution containing lactate,
   wherein the lactate is selected from sodium lactate or potassium lactate in an amount of 5-100 mM, for example, 5-75 mM, 5-50 mM, or 10-100 mM, preferably 5-50 mM;
(2) adding a lyoprotectant; and
(3) lyophilizing to give a lyophilized formulation of the nucleic acid-lipid nanoparticle composition.

The nucleic acid, the lipid, the nucleic acid-lipid nanoparticle, the nucleic acid-lipid nanoparticle composition, and the lyophilized formulation are as defined herein.

In some embodiments, the lyoprotectant is one or more selected from the group consisting of sucrose, trehalose, lactose, cyclodextrin, and mannitol.

In some embodiments, the content of the lyoprotectant is 50-99.9 wt%, for example, 50%, 55%, 60%, 65%, 70%, 75%, 80%, 85%, 90%, or 95%, for example, a range with any two of the values in the above ranges as endpoints and a value in the range, on dry basis by the weight of the lyophilized formulation.

In some embodiments, step (1) further comprises adding a buffer, the content ratio of the buffer to the lactate being (0.25-1):1.

In some embodiments, the buffer is one selected from or a combination of more selected from the group consisting of an acetate buffer, a PBS buffer, a citrate buffer, a phosphate buffer, a histidine buffer, and a tris buffer. The content of the buffer is 1.25-100 mM, for example, 2.5-100 mM, 5-100 mM, 5-75 mM, or 5-50 mM; preferably, the content of the buffer is 1.25-50 mM or 5-50 nM.

In some embodiments, the pH of the solution prepared in step (1) is 1.0-14.0, for example, 1.0, 2.0, 3.0, 4.0, 5.0, 6.0, 7.0, 8.0, 9.0, 10.0, 11.0, 12.0, 13.0, 14.0, and a range with any two of the above values as endpoints and a value in the range, for example, 2.0-13.0, 3.0-12.0, 4.0-11.0, 5.0-10.0, 5.0-9.0, 6.0-9.0, 6.0-8.0, 6.0-7.0, and 7.0-8.0. In some embodiments, the pH of the nucleic acid-lipid nanoparticle composition is 7.0, 7.1, 7.2, 7.3, 7.4, 7.5, 7.6, 7.7, 7.8, 7.9, 8.0, or a range with any two of the above values as endpoints and a value in the range.

In some embodiments, the method of preparing the nucleic acid-lipid nanoparticle composition further comprises: after the preparation is completed, conducting a sterile filtration, and filling the filtrate sample into a vial for lyophilization. In some embodiments, after half-stoppering with a lyophilization rubber stopper, the vail is loaded on a lyophilizer for lyophilization according to a preset program. In some embodiments, the filter is 0.22 µm.

In some embodiments, the lyophilization comprises: a pre-freezing stage, a primary drying stage, and a desorption drying stage.

For the pre-freezing stage, the temperature is -80 °C to -30 °C, for example, -80 °C, -70 °C, - 60 °C, -50 °C, -40 °C, -30 °C, or a range with any two of the above values as endpoints and a value in the range; the pre-freezing time is 1-6 hours, for example, 1 hour, 2 hours, 3 hours, 4 hours, 5 hours, 6 hours, or a range with any two of the above values as endpoints and a value in the range.

For the primary drying stage (which may also be referred to as a sublimation drying stage): the temperature is -60 °C to 0 °C, for example, -60 °C, -50 °C, -40 °C, -30 °C, -20 °C, -10 °C, 0 °C, or a range with any two of the above values as endpoints and a value in the range; the endpoint temperature is -5 °C to 0 °C, for example, -5 °C, -4 °C, -3 °C, -2 °C, -1 °C, 0 °C, or a range with any two of the above values as endpoints and a value in the range; the vacuum pressure is controlled at ≤ 10 Pa; the duration time of the primary drying stage is 5 to 100 hours, for example, 5 hours, 10 hours, 20 hours, 30 hours, 40 hours, 50 hours, 60 hours, 70 hours, 80 hours, 90 hours, 100 hours, or a range with any two of the above values as endpoints and a value in the range. In some embodiments, the temperature of the primary drying stage is gradually increased in 2-8 stages. In each stage, the temperature is increased by 5-30 °C, the duration is 0.5-10 hours, and the vacuum pressure is controlled at ≤ 10 Pa.

For the desorption drying stage: the temperature is 0 °C to 35 °C, for example, 0 °C, 5 °C, 10 °C, 15 °C, 20 °C, 25 °C, 30 °C, 35 °C, or a range with any two of the above values as endpoints and a value in the range; the endpoint temperature is 30 °C to 35 °C, for example, 30 °C, 31 °C, 32 °C, 33 °C, 34 °C, 35 °C, or a range with any two of the above values as endpoints and a value in the range; the vacuum pressure is controlled at ≤ 10 Pa; the duration time of the desorption drying stage is 2 to 15 hours, for example, 2 hours, 3 hours, 4 hours, 5 hours, 6 hours, 7 hours, 8 hours, 9 hours, 10 hours, 11 hours, 12 hours, 13 hours, 14 hours, 15 hours, or a range with any two of the above values as endpoints and a value in the range.

### Use and Administration

The present disclosure provides use of the nucleic acid-lipid nanoparticle composition disclosed herein in preparing a medicament.

In some embodiments, the medicament is a vaccine.

In some embodiments, the vaccine is a therapeutic tumor vaccine or a vaccine for preventing a cancer, a viral infection, a bacterial infection, or a fungal infection.

In some embodiments, the virus is selected from the group consisting of norovirus, Ebola virus, coronavirus, cytomegalovirus, Dengue virus, Zika virus, enterovirus, hepatitis virus, herpes simplex virus, human papilloma virus, influenza virus, Marburg virus, measles virus, poliovirus, rabies virus, and rotavirus.

In some embodiments, the medicament is a formulation for oral administration, intramuscular injection, subcutaneous injection, intravenous injection, or inhalation.

In some embodiments, the formulation for inhalation is an atomized inhalant or a dry powder inhalant.

### Examples

The embodiments of the present disclosure will be clearly and completely described below with reference to the examples, and it is obvious that the described examples are some of the examples of the present disclosure but not all of them. Based on the examples of the present disclosure, all other examples obtained by those of ordinary skill in the art without creative work shall fall within the protection scope of the present disclosure.

The embodiments and beneficial effects of the present disclosure will be further described below with reference to preferred examples.

### Example 1. Exploration on identity of lactates

### Modeling samples:

mRNA encoding herpes zoster virus gE protein (gE-mRNA, about 2300 bp) was used as the modeling mRNA, and an mRNA lipid nanoparticle (mRNA-LNP) without the addition of sodium lactate and lyophilization was used as the modeling sample.

The preparation process is as follows: an mRNA stock solution was diluted to 135 µg/mL with sodium acetate buffer, and a lipid mixture solution was prepared in a molar ratio of cationic lipid ALC-0315:DSPC:cholesterol:DMG-PEG2000 = 49:10:39.5:1.5; after the encapsulation was completed on an instrument, the solution was subjected to buffer exchange by ultrafiltration, concentration, and sterile filtration through a 0.22 µm filter to give the mRNA-LNP stock solution.

In order to further explore the mRNA-LNP lyophilization effects of lactates, stock solutions of different lactates were added into the mRNA-LNP in addition to sodium lactate, and the lyophilization effects of potassium lactate, ammonium lactate, lactic acid, lithium lactate, magnesium lactate, and calcium lactate were determined and compared. In Examples 1-1 to 1-7, the amounts of the lactates were 20 mM, while in Example 1-8, an mRNA-protamine complex with sodium lactate was added (the mRNA-protamine complex was prepared by preparing mRNA into an aqueous solution of 0.4 g/L, mixing with protamine in an mRNA/protamine ratio of 4:1 (w/w), adding a sodium lactate stock solution and a lyoprotectant stock solution, diluting to an mRNA concentration of about 100 µg/mL, a sodium lactate concentration of 20 mM, and a lyoprotectant concentration of at least 5% (w/v); the lyoprotectant was selected from one or more of sucrose, trehalose, mannitol, glucose, maltose, and lactose), and the mRNA-LNP and mRNA-protamine complex were separately lyophilized and assessed.

The lyoprotectant stock solution was added to the stock mRNA-LNP solution. After the preparation, the samples were subjected to sterile filtration through a 0.22 µm filter, and the filtrate samples were filled into 2R vials at 0.6 mL/vial. The vials were half-stoppered with a lyophilization rubber stopper and loaded on an SP Scientific VirTis Genesis 25L Pilot Lyophilizer for lyophilization according to a preset program (the samples were pre-freezed at -55 °C for 3 hours, warmed to -20 °C within 50 min, subjected to the primary drying for 10 hours, and heated to 30 °C at the maximum temperature ramping rate for 10 hours of desorption drying; the pressure in the primary drying stage and the secondary drying stage was controlled at 50 mTorr).

**Table 1. Lyophilization effect of different lactates**

| Sample name | Amount | Salts | Appearance after lyophilization | Particle size (nm) | mRNA content (µg/mL) | Encapsulation efficiency (%) | Integrity (%) |
|---|---|---|---|---|---|---|---|
| mRNA-LNP stock solution before lyophilization | / | / | / | 87.6 | 97 | 95.6 | 93.1 |
| Example 1-1 | 20mM | Sodium lactate | No defects in appearance | 94.5 | 95 | 90.9 | 93.7 |
| Example 1-2 | 20mM | Potassium lactate | No defects in appearance | 95.8 | 96 | 90.1 | 92.6 |
| Example 1-3 | 20mM | Ammonium lactate | No defects in appearance | 96.3 | 94 | 76.6 | 69.2 |
| Example 1-4 | 20mM | Lactic acid | No defects in appearance | 98.7 | 88 | 95.5 | 60.0 |
| Example 1-5 | 20mM | Lithium lactate | No defects in appearance | 96.8 | 98 | 88.4 | 50.2 |
| Example 1-6 | 20mM | Calcium lactate | No defects in appearance | 103.9 | 90 | 83.9 | 45.8 |
| Example 1-7 | 20mM | Magnesium lactate | No defects in appearance | 108.4 | 87 | 85.0 | 42.7 |
| Example 1-8 (protamine-mRNA complex) | 20mM | Sodium lactate | Complete collapse | 225.4 | 85 | 40.4 | 82.5 |

The RiboGreen RNA reagent and the free mRNA were combined for fluorescence excitation, and the amount of free mRNA was determined by a fluorescence analyzer; meanwhile, the amount of total mRNA was measured by membrane lysis with Triton X-100, then: Encapsulation efficiency (EE%) = (amount of total mRNA - amount of free mRNA)/amount of total mRNA × 100%

Determination of mRNA integrity: The mRNA-LNP sample was diluted and denatured at 70 °C, Dye-Gel was prepared and injected into a gel pressing device, and then the mRNA marker, ladder, and sample were added separately. The mRNA integrity spectrogram of the samples was obtained by gel electrophoresis on an Agilent 5200 bioanalysis system.

As can be seen from Table 1, the results of Examples 1-1 to 1-7 show that potassium lactate and sodium lactate have good lyophilization effects, but considering that in some special dosage forms, for example, the mainstream mRNA-LNP dosage form injection, the higher potassium ion concentration after injection will affect the myocardial cell electrophysiology, thus imposing a certain safety risk probability. Therefore, in the case that potassium lactate and sodium lactate can achieve good lyophilization effects, sodium lactate may be preferred. Also, the results of Example 1-8 show that sodium lactate used in the protamine-mRNA complex may lead to complete collapse after lyophilization with low encapsulation efficiency and integrity.

The prior art holds the recognition that the introduction of salts can adversely effect the lyophilization of lipid nanoparticle formulations, whereas the present disclosure breaks through the limitation of conventional studies by adding lactates, and proves the performance of lactates in lyophilization by electron microscopy on the samples of Example 1-1.

On a graphene carrier net, the sample was placed on a watch glass with the front side facing upwards, and detected on a glow discharge spectrometer for 2 seconds (the detection was manually stopped). The lyophilized samples were prepared, 1) the lyophilizer was started half an hour in advance, the filter paper was replaced, double distilled water was added into the lyophilizer, and the lyophilization parameters of Blot Time (2.5 s), Blot Force (3 s), Wait Time (3 s) and Blot Total (1 s) were input for sample preparation.

Liquid nitrogen and liquid ethane were added into a mold. The carrier net was clamped on a clamp according to the direction of the clamping groove, and then the clamp and the carrier net were mounted on the lyophilizer; 3 µL of the reconstituted sample of Example 1-1 was carefully transferred to the surface of the carrier net by a pipette (care was taken to prevent the pipette tip from puncturing the carrier net), and the instrument was started according to the sample preparation conditions; the clamp was removed, and the carrier net was quickly transferred from ethane to liquid nitrogen (care was taken to prevent net from bending) and mounted in an electron microscopic rotating wheel die. After the samples were preserved, the lyophilization was completed.

The frozen carrier net was loaded on an FEI 200KV Talos Arctica G2 field emission transmission electron microscope, and the sample was cooled and loaded for electron microscopy and photography.

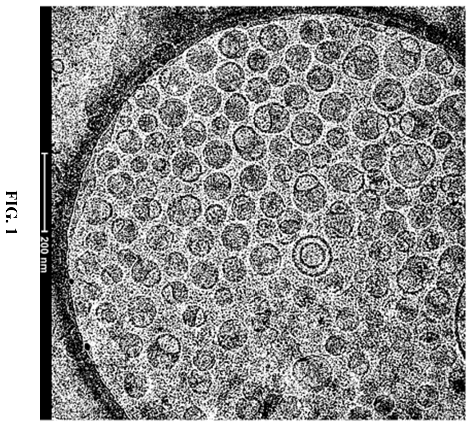

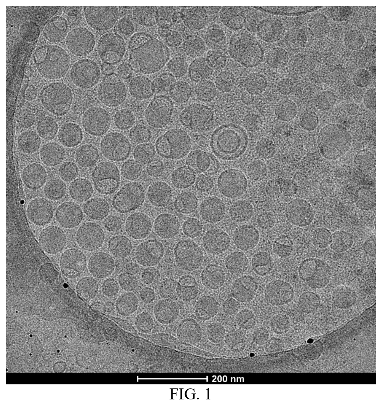

From the cryogenic electron microscopic results of the samples of Example 1-1, it can be seen that even with the use of sodium lactate at a high concentration (20 mM) for lyophilization, the formulation after lyophilization and reconstitution still exhibited a uniform spherical shape, suggesting that the addition of sodium lactate for lyophilization did not significantly change the micro-morphology of mRNA-LNP (FIG. 1).

### Example 2. Exploration on sodium lactate alone and in combination

**Table 2. Lyophilization buffer composition**

| Sample name | Buffer composition |
|---|---|
| Example 2-1 | pH8.0 (5mM Tris) |
| Example 2-2 | pH 7.5 (20 mM sodium lactate) |
| Example 2-3 | pH 7.5 (20 mM Tris + 5 mM sodium lactate) |

mRNA-LNP stock solutions having an mRNA concentration of about 200 µg/mL but no buffer were prepared according to the modeling sample preparation method in Example 1. An appropriate volume of the mRNA-LNP stock solution was added to an appropriate volume of 100 mM Tris stock solution (pH 7.5), Tris stock solution (pH 8.0), or sodium lactate stock solution (pH 7.5), and then appropriate volumes of ultrapure water and the lyoprotectant stock solution were added to dilute the mRNA to about 100 µg/mL. The final buffer compositions are shown in Table 2. After the preparation, samples 1-3 were subjected to sterile filtration through a 0.22 µm filter, and the filtrate samples were filled into 2R vials at 0.6 mL/vial. The vials were half-stoppered with a lyophilization rubber stopper and loaded on a lyophilizer for lyophilization according to a preset program.

After the lyophilization was completed, nitrogen was introduced, and the vials were stoppered, taken out from the lyophilization cabinet, and was capped. 0.54 mL of enzyme-free water was added to the lyophilized sample for reconstitution, and the time for reconstitution was recorded. An appropriate amount of the reconstituted sample was 50-fold diluted and detected for particle size and polydispersity index on a Malvern particle size analyzer. The results were the means of three parallel measurements, and the pH, the mRNA content, the encapsulation efficiency, and the integrity of the reconstituted sample were determined. The results are shown in Table 3.

**Table 3. Detection results of samples with different lyophilization buffers**

| Sample name | Average particle size (nm) | PDI | mRNA content (µg/mL) | Encapsulation efficiency (%) | Integrity (%) | pH after reconstitution | Reconstitution time (s) |
|---|---|---|---|---|---|---|---|
| Example 2-1 | 98.28 | 0.113 | 91 | 88 | 80.9 | 7.98 | 15 |
| Example 2-2 | 98.40 | 0.146 | 86 | 87 | 96.0 | 7.31 | 12 |
| Example 2-3 | 97.53 | 0.108 | 100 | 88 | 97.8 | 7.48 | 11 |

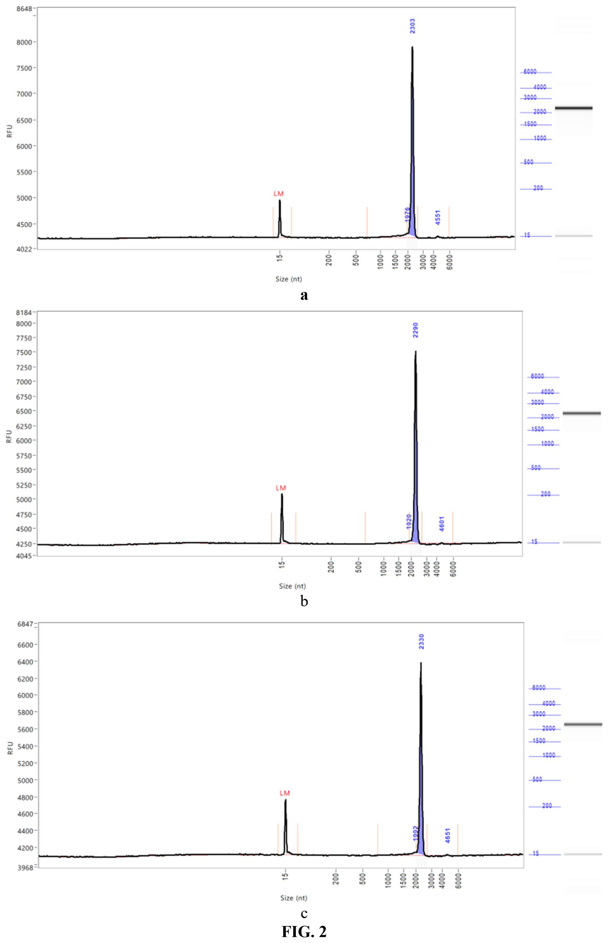

The integrity determination profiles of samples 1-3 in Example 2 are shown in FIG. 2.

100 µL of the mRNA-LNP samples after lyophilization and reconstitution was taken and added into a 0.6-mL centrifuge tube for pH measurement. The pH meter was Mettler Toledo FE28 with a micro pH probe.

The results show that the formulations after lyophilization can be quickly reconstituted. The particle size of the reconstituted mRNA-LNP formulations was still in a reasonable range with a small PDI, indicating that the particle size distribution is centralized. The mRNA encapsulation efficiency and integrity were at high levels, suggesting that the sample was successfully lyophilized.

To distinguish the stability differences of samples 1-3 in Example 2 in a short time, the lyophilized formulations of samples 1-3 were placed at 42 °C for one month, and the particle size, polydispersity index, pH, mRNA content, encapsulation efficiency, and integrity were measured after reconstitution at weeks one, two, three, and four. The results are shown in Table 4.

**Table 4. Detection results at different time points after exposure to a temperature of 42 °C**

| Sample name | Time point | Average particle size (nm) | PDI | mRNA content (µg/mL) | Encapsulation efficiency (%) | Integrity (%) |
|---|---|---|---|---|---|---|
| Example 2-1 | Week 1 | 110.50 | 0.129 | 91.0 | 88.7 | 68.4 |
| | Week 2 | 124.80 | 0.122 | 87.4 | 86.8 | 44.1 |
| | Week 3 | 121.23 | 0.081 | 93.5 | 85.6 | 49.8 |
| | Week 4 | 145.21 | 0.102 | 86.7 | 82.1 | 30.6 |
| Example 2-2 | Week 1 | 144.80 | 0.064 | 96.8 | 90.2 | 94.8 |
| | Week 2 | 154.80 | 0.094 | 95.2 | 86.4 | 79.5 |
| | Week 3 | 158.10 | 0.091 | 94.5 | 86.0 | 78.4 |
| | Week 4 | 163.80 | 0.095 | 91.2 | 81.9 | 71.7 |
| Example 2-3 | Week 1 | 136.80 | 0.063 | 95.0 | 86.6 | 62.1 |
| | Week 2 | 136.00 | 0.079 | 98.1 | 84.5 | 39.4 |
| | Week 3 | 146.60 | 0.091 | 88.6 | 81.8 | 31.6 |
| | Week 4 | 141.34 | 0.105 | 93.6 | 80.4 | 23.3 |

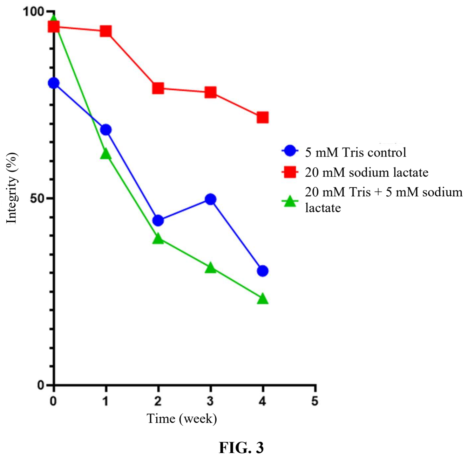

As can be seen from Table 4, after the samples were exposed to a temperature of 42 °C, the particle size of samples 1-3 was increased to a certain extent, the encapsulation efficiency was reduced to a small extent, the mRNA content was kept stable, and the integrity was greatly differentiated. As shown in FIG. 3, after the samples were exposed to 42 °C for one month, the integrity of the 5 mM Tris group and the 20 mM Tris + 5 mM sodium lactate group was reduced to about 30%, and the change trend was close to that reported by Muramatsu et al. Unexpectedly, however, the samples in the 20 mM sodium lactate group had an integrity of up to 71.7% even after exposure to 42 °C for one month, which was significantly higher than the other two groups, suggesting that the use of 20 mM sodium lactate can significantly improve the reduction of the integrity of the lyophilized formulation.

In this study, Example 2-2 showed that the addition of sodium lactate to the nucleic acid-lipid nanoparticle formulation achieved the technical effect of improving the integrity and long-term storage stability of the lyophilized nucleic acid-lipid nanoparticle formulation, but also exhibited a pH of 7.31 that is lower than the target pH 7.5 with a remarkable fluctuation during the pH detection. This may be correlated with the weak buffering capacity of sodium lactate at pH 7-8. Therefore, in order to provide better technical effects, a small amount of Tris was further added to different amounts of sodium lactate to improve the pH buffering capacity of the formulation so as to protect mRNA from pH-fluctuation degradation, thereby improving the integrity and stability of the lyophilized formulation.

**Table 5. Formulas of Tris and sodium lactate combinations**

| Sample name | Compositions of buffer combinations |
|---|---|
| Example 2-4 | 5 mM Tris & 5 mM sodium lactate, pH 7.5 |
| Example 2-5 | 5 mM Tris & 10 mM sodium lactate, pH 7.5 |
| Example 2-6 | 5 mM Tris & 20 mM sodium lactate, pH 7.5 |

mRNA-LNP stock solutions having an mRNA concentration of about 200 µg/mL with 5 mM Tris as the buffer were prepared according to the modeling sample preparation method. An appropriate volume of the mRNA-LNP stock solution was added to an appropriate volume of 100 mM sodium lactate stock solution, and then appropriate volumes of 5 mM Tris solution and the lyoprotectant stock solution were added to dilute the mRNA to about 100 µg/mL. The final buffer compositions are shown in Table 5. After the preparation, samples 4-6 were subjected to sterile filtration through a 0.22 µm filter, and the filtrate samples were filled into 2R vials at 0.6 mL/vial. The vials were half-stoppered with a lyophilization rubber stopper and loaded on a lyophilizer for lyophilization according to a preset program.

After the lyophilization was completed, nitrogen was introduced, and the vials were stoppered, taken out from the lyophilization cabinet, and was capped. 0.54 mL of enzyme-free water was added to the lyophilized sample for reconstitution, and the time for reconstitution was recorded. An appropriate amount of the reconstituted sample was 50-fold diluted and detected for particle size and polydispersity index on a Malvern particle size analyzer. The results were the means of three parallel measurements, and the mRNA content, the encapsulation efficiency, and the integrity of the reconstituted sample were determined. The results are shown in Table 6.

**Table 6. Critical quality attributes and reconstitution time after lyophilization**

| Sample name | Average particle size (nm) | PDI | mRNA content (µg/mL) | Encapsulation efficiency (%) | Integrity (%) | pH before lyophilization | pH after reconstitution | Integrity after reconstitution following four-week exposure to 42 °C | pH after reconstitution following four-week exposure to 42 °C | Reconstitution time (s) |
|---|---|---|---|---|---|---|---|---|---|---|
| Example 2-4 | 106.6 | 0.15 | 89 | 90.8 | 94 | 7.54 | 7.42 | 82 | 7.45 | 15 |
| Example 2-5 | 99.88 | 0.0966 | 92 | 89.4 | 89.8 | 7.39 | 7.48 | 79 | 7.44 | 14 |
| Example 2-6 | 99.52 | 0.111 | 90 | 87.3 | 92 | 7.4 | 7.47 | 81 | 7.42 | 16 |

100 µL of the mRNA-LNP sample after lyophilization and reconstitution and the sample before lyophilization were taken and added into a 0.6-mL centrifuge tube for pH measurement. The pH meter was Mettler Toledo FE28 with a micro pH probe.

The RiboGreen RNA reagent and the free mRNA were combined for fluorescence excitation, and the amount of free mRNA was determined by a fluorescence analyzer; meanwhile, the amount of total mRNA was measured by membrane lysis with Triton X-100, then: Encapsulation efficiency (EE%) = (amount of total mRNA - amount of free mRNA)/amount of total mRNA × 100%

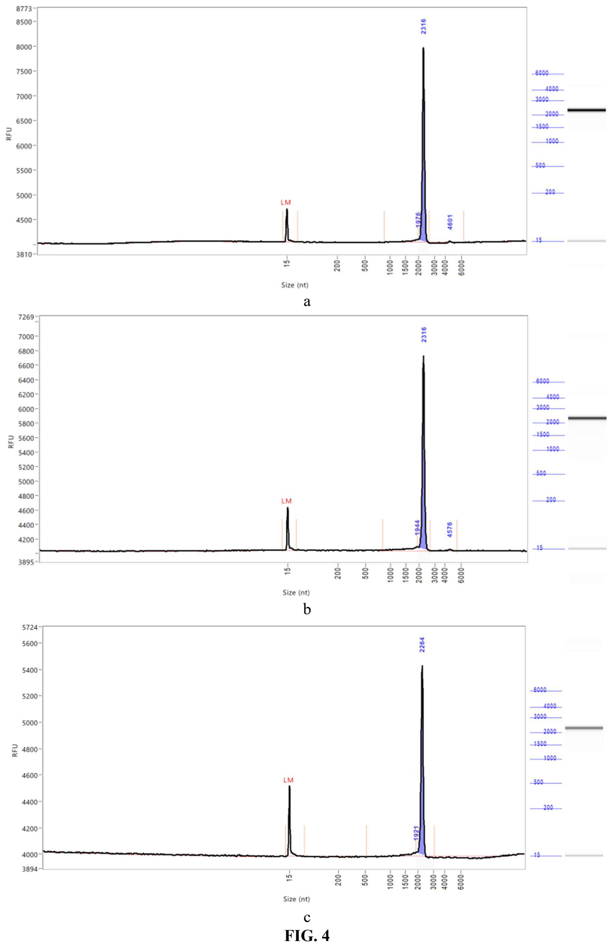

Determination of mRNA integrity: The mRNA-LNP sample was diluted and denatured at 70 °C, Dye-Gel was prepared and injected into a gel pressing device, and then the mRNA marker, ladder, and sample were added separately. The mRNA integrity spectrogram of the samples was obtained by gel electrophoresis on an Agilent 5200 bioanalysis system. The integrity determination profiles of samples in Examples 2-4, 2-5, and 2-6 are shown in FIG. 4.

The results show that the formulations of the Tris and sodium lactate combination after lyophilization can be quickly reconstituted. The particle size of the reconstituted mRNA-LNP formulations was still in a reasonable range with a small PDI, indicating that the particle size distribution is centralized. The mRNA encapsulation efficiency and integrity were at high levels, suggesting that the sample was successfully lyophilized. From the pH before and after lyophilization, the combination of Tris and sodium lactate stabilized the pH of the mRNA-LNP sample before and after lyophilization to a level very close to the target pH 7.5, thus preventing the mRNA from being degraded due to the pH fluctuation.

### Western-Blot of Expression in Cells

HEK293 cells were used as the *in-vitro* protein expression model cells. The cells were plated in 6-well plates at a cell density of 1 × 10⁶ cells/mL and cultured for 24 hours. Then the samples of Examples 2-1 to 2-6 were reconstituted in nuclease-free water and added to the wells in sequence at an amount of 20 µL along with the modeling sample. After mixing, the system was incubated for 4 hours for transfection, and the medium (containing serum) was exchanged.

After 24 hours of incubation in an incubator, the cells were digested with PBS, transferred to a centrifuge tube, and centrifuged (2500 r/min, 5 min). The cells were collected, resuspended in 1 mL of PBS, transferred to a 1.5-mL EP tube, and centrifuged. The cells in the precipitate were collected. 200 µL of a protease lysis buffer (1 µL protease inhibitor per 100 µL CHAPS) was added to the samples, and the cells were lysed on ice for 1 hour. The mixture was centrifuged at 4 °C in a centrifuge (13500 r/min, 20 minutes), and the supernatant was collected. The samples were subjected to Western-Blot assay to determine the protein expression level.

Firstly, the electrophoresis device was subjected to a leak test (the electrophoresis device was filled with water, and no level drop within 5 min indicates the acceptance of the electrophoresis device), and the water was removed after the leak test was completed. A prepared separation gel was added at 12% (v/v) to the electrophoresis device before absolute ethanol was added (for removing bubbles). The system was stood for 30 min to solidify the separation gel. The absolute ethanol was poured, and the residual liquid was removed by filter paper. The comb was inserted soon after the concentration gel was added. The system was stood for 30 min for solidification. The device was placed into the electrophoresis tank, and the electrophoresis solution (1× SDS-PAGE buffer solution) to submerge the platinum wire. The comb was removed, and samples 1-3 in Example 1, samples 4-6 in Example 3, and the protein expression sample of the model sample were added in sequence. The electrophoresis (100 V, 300 mA) was performed. The positions of bromophenol blue and the marker were monitored in the electrophoresis process, and the electrophoresis was stopped when bromophenol blue and the marker reached the end of the gel. The gels were taken out after electrophoresis, and the concentration gel and separation gel were separated according to the boundary therebetween. The concentration gel was discarded and the separation gel was reserved. The separation gel and a filter paper were soaked in 1× blotting buffer, and a PVDF membrane was soaked in absolute methanol for activation. 3 filter papers, 1 PVDF membrane, separation gel, and another 3 filter papers were sequentially placed on a membrane transfer device, and the bubbles were removed. The device was started and operated at 15 V (gradually increased from 5 V to 15 V) and 300 mA for transferring for 1 hour and 40 minutes. The PVDF membrane (with protein) was soaked in 1× PBST containing 5% of skimmed milk powder, blocked at room temperature for 2 hours, and washed to remove excessive skimmed milk powder with 1× PBST after the 2 hours. The membrane was transferred in a 15-mL centrifuge tube containing a primary antibody (diluted with 5% skimmed milk powder as per the instruction), incubated overnight at 4 °C, and washed with 1× PBST to remove excess primary antibody. The PVDF membrane with the primary antibody blocked was placed into a 15-mL centrifuge tube containing a secondary antibody (diluted with 5% skim milk powder according to the instruction), incubated for 1 hour at room temperature (away from light), washed to remove excess secondary antibody, and detected and analyzed on an Odyssey infrared laser imaging system.

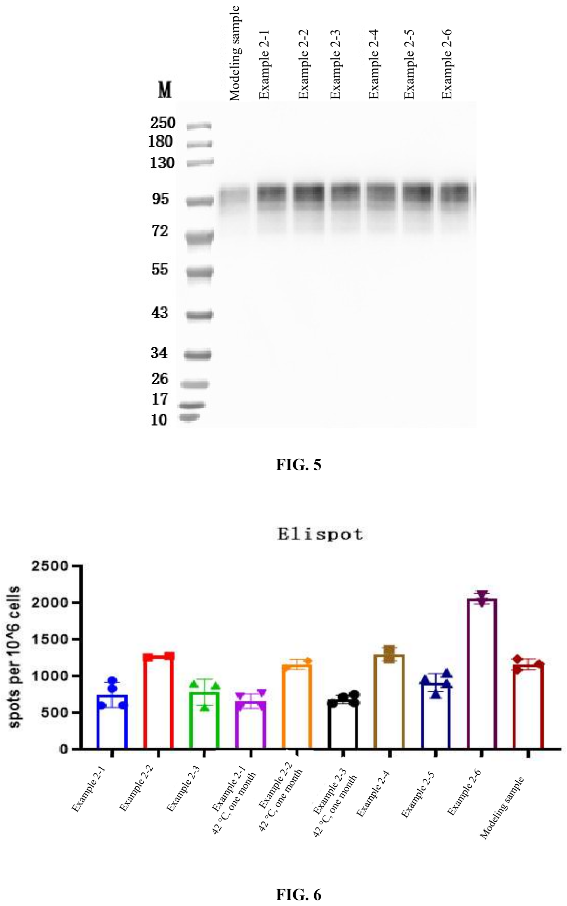

The results are shown in FIG. 5. It can be seen that the expression levels of gE protein in the samples of Examples 2-1 to 2-6 in Example 2 demonstrated no significant difference, and were even higher than those of the modeling samples that were not lyophilized. The cell transfection efficiency in samples of all groups demonstrated no significant difference, indicating that the intracellular biological activity of the mRNA-LNP is not reduced by the lyophilization process, and the intracellular biological activity of the mRNA-LNP is not significantly influenced by the use of sodium lactate alone or in different amounts in combination with Tris.

### Immunization in Mice

The study compared 9 reconstituted formulations (including three formulations of Examples 2-1 to 2-3, three formulations of Examples 2-1 to 2-3 accelerated at 42 °C for four weeks, and three formulations of Examples 2-4 to 2-6) with the non-lyophilized modeling sample. BALB/c mice were randomized into 10 groups and injected intramuscularly with 100 µL (5 µg) of the samples on days 0 and 14. Blood samples were collected on days 14 and 28, and subjected to IgG detection and alternative neutralizing antibody detection for the VZV gE protein. Splenocytes were collected for ICS assay for the gE protein antigen specificity. The samples were combined and PBMCs were collected for ELISPOT for the gE protein antigen specificity.

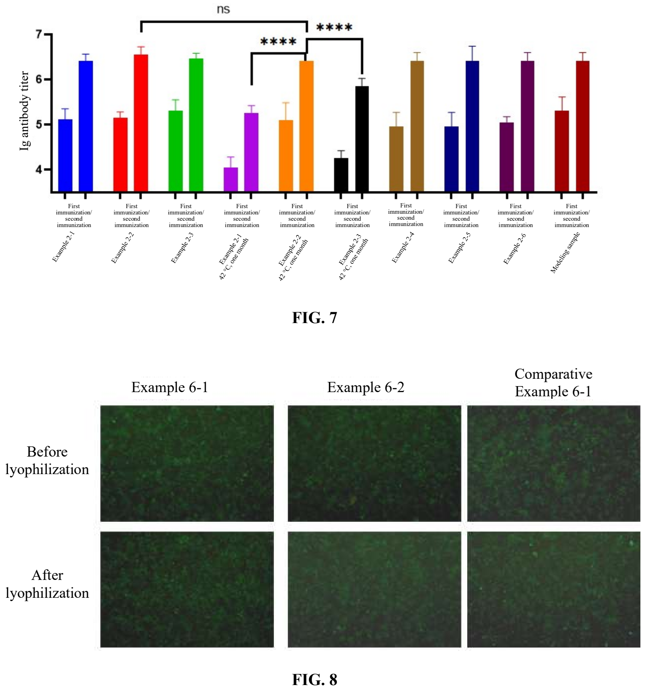

As can be seen from the cellular immunity and the humoral immunity levels in FIGs. 6 and 7, the humoral immunity and the cellular immunity levels of the lyophilized formulations were close to those of the modeling sample before lyophilization. The lyophilization process does not lead to a significant decrease in the *in-vivo* immune response in mice, with some of the lyophilized formulations even showing significantly higher *in-vivo* immunization activity than that of the modeling formulation before lyophilization. In addition, it can be found that the *in-vivo* immunization activities of different samples in mice were significantly differentiated after the lyophilized formulations were exposed to 42 °C for 4 weeks. The *in-vivo* immunization activity in the mice of the sodium lactate-containing formulation after the 4-week exposure to 42 °C exhibited no significant decrease as compared to the baseline before lyophilization, while those of the other two groups of lyophilized samples demonstrated significant decreases after the 4-week exposure to 42 °C, which corresponds to the integrity results after the lyophilized formulations were exposed to 42 °C for 4 weeks.

**Table 7. Samples for mouse immunization**

| No. | Formula information | Sample |
|---|---|---|
| Gr1 | pH8.0 (5mM Tris) | Example 2-1 |
| Gr2 | pH 7.5 (20 mM sodium lactate) | Example 2-2 |
| Gr3 | pH 7.5 (20 mM Tris + 5 mM sodium lactate) | Example 2-3 |
| Gr4 | pH8.0 (5mM Tris) | Example 2-1, 42 °C for four weeks |
| Gr5 | pH 7.5 (20 mM sodium lactate) | Example 2-2, 42 °C for four weeks |
| Gr6 | pH 7.5 (20 mM Tris + 5 mM sodium lactate) | Example 2-3, 42 °C for four weeks |
| Gr7 | 5 mM Tris & 5 mM sodium lactate, pH 7.5 | Example 2-4 |
| Gr8 | 5 mM Tris & 10 mM sodium lactate, pH 7.5 | Example 2-5 |
| Gr9 | 5 mM Tris & 20 mM sodium lactate, pH 7.5 | Example 2-6 |
| Gr10 | Frozen liquid formulation before lyophilization (pH 7.5, 20 mM Tris) | Modeling sample before lyophilization |

### Example 3. Exploration on amount of sodium lactate

In order to examine the effect of different amounts of sodium lactate on the lyophilization of the LNP formulation, LNP formulations of different sodium lactate concentrations were prepared and lyophilized according to the preparation method and lyophilization procedures in Example 1. The specific lyophilization results are shown in Table 8.

**Table 8. Exploration on amount of sodium lactate**

| Sample name | Amount of sodium lactate (mM) | Appearance after lyophilization | Particle size (nm) | mRNA content (µg/mL) | Encapsulation efficiency (%) | Integrity (%) | pH | Theoretical mass fraction (%) of sodium lactate in lyophilized solid² |
|---|---|---|---|---|---|---|---|---|
| mRNA-LNP stock solution before lyophilization | / | / | 84.7 | 84 | 96.2 | 92.7 | / | / |
| Comparative Example 3-1¹ | 0 | No defects in appearance | 95.6 | 85 | 90 | 93.1 | 7.54 | 0 |
| Example 3-1 | 5 | No defects in appearance | 96.8 | 86 | 92.1 | 95.6 | 7.33 | 0.37 |
| Example 3-2 | 10 | No defects in appearance | 96.2 | 84 | 92.6 | 89.2 | 7.23 | 0.74 |
| Example 3-3 | 20 | No defects in appearance | 98.4 | 81 | 90.5 | 90.0 | 7.33 | 1.47 |
| Example 3-4 | 30 | No defects in appearance | 117.2 | 88 | 88.4 | 90.2 | 7.27 | 2.19 |
| Example 3-5 | 40 | No defects in appearance | 101.7 | 86 | 88.7 | 90.0 | 7.27 | 2.90 |
| Example 3-6 | 50 | No defects in appearance | 98.4 | 80 | 86.0 | 92.7 | 7.27 | 3.60 |
| Example 3-7 | 75 | No defects in appearance | 105.4 | 85 | 80.4 | 92.5 | 6.92 | 5.30 |
| Example 3-8 | 100 | Slight shrinkage | 121.3 | 83 | 75.2 | 81.3 | 7.03 | 6.95 |
| Example 3-9 | 125 | Complete collapse | 143.8 | 83 | 71.3 | 75.3 | 7.11 | 8.54 |
| Example 3-10 | 150 | Complete collapse | 167.5 | 80 | 68.7 | 77.5 | 7.09 | 10.07 |

| | | | | | | | | |
|---|---|---|---|---|---|---|---|---|
| ¹ Comparative Example 3-1 served as a sodium lactate-free control; 20 mM Tris buffer pH 7.5 (the buffer formulation of commercially available mRNA-1273 from Modema corporation) was added and the product was lyophilized. ² The theoretical calculation was on a dry basis where water was completely removed after lyophilization. The solid matters after lyophilization were the lyoprotectant, the mRNA-LNP, and sodium lactate, and the theoretical mass fraction (%) of sodium lactate in the lyophilized solid was based on the sum of the masses of the lyoprotectant, the mRNA-LNP, and sodium lactate. | | | | | | | | |

As can be seen from the lyophilization results of Examples 3-1 to 10, when the amount of sodium lactate was 100 mM or greater, certain defects were observed in terms of the appearance of the lyophilized cake, and particularly, when the amount was 125 mM or greater, the cake completely collapsed. For the encapsulation efficiency of the lyophilized formulations, when the amount of sodium lactate was 50 mM or greater, the encapsulation efficiency after lyophilization was significantly reduced along with the increase in the amount of sodium lactate, and particularly when the amount of sodium lactate was 100 mM or greater, the encapsulation efficiency after lyophilization was less than 80%. From the integrity, due to certain errors of the detection method, it can be considered that when the amount of sodium lactate was 5-75 mM, the integrities of the lyophilized formulations were at high levels and exhibited no significant decrease. When the amount of sodium lactate reached a concentration of 100 mM or greater, the integrity after lyophilization was significantly reduced, which corresponds to the reduction in encapsulation efficiency after lyophilization. Meanwhile, the certain difference between the pH value after lyophilization and reconstitution and the preset pH value 7.5 before lyophilization was noticed, and especially when the amount of sodium lactate was 75 mM or greater, the pH value after lyophilization and reconstitution was reduced to about 7, which may be related to the weak buffering capacity and the near neutrality of a sodium lactate solution.

On the basis of the preparation of lyophilized formulations containing different amounts of sodium lactate, to further confirm the thermal stability of the lyophilized formulations containing different amounts of sodium lactate, an influencing factor study of a high temperature 42 °C was conducted. The integrity was determined in samples after a two- or four-week exposure to a high temperature of 42 °C, and the specific results are shown in Table 9. The integrities of the Comparative Example 3-1 and Example 3-1 decreased to around 30% after the 4-week exposure to 42 °C, while the integrities of the remaining samples remained between 73-83% after the four-week exposure to 42 °C and were close to the result of Example 2-2.

The integrity results of samples of Examples 2-2/2-3 after the four-week exposure to 42 °C suggested that 5 mM amount of sodium lactate possesses limited protective effects on the mRNA, the integrity of the lyophilized formulation containing 5 mM of sodium lactate after the four-week exposure to 42 °C exhibited a similar decrease to that of the formulation with no sodium lactate, and was much less than those with 10 mM or greater amount of sodium lactate, which may be related to the mechanism of sodium lactate protecting mRNA from degradation in high-temperature conditions. Sodium lactate protects the mRNA by reducing the activity of residual water in the lyophilized formulations, and thus a lower amount of sodium lactate may not completely reduce the activity of residual water in the lyophilized formulations.

**Table 9. Exploration on amount of sodium lactate after exposure to 42 °C**

| Sample name | Amount of sodium lactate (mM) | Baseline integrity (%) | Integrity after 2-week exposure to 42 °C (%) | Integrity after 4-week exposure to 42 °C (%) |
|---|---|---|---|---|
| Comparative Example 3-1 | 0 | 93.1 | 37.2 | 30.3 |
| Example 3-1 | 5 | 95.6 | 39.7 | 28.4 |
| Example 3-2 | 10 | 89.2 | 81.3 | 77.9 |
| Example 3-3 | 20 | 90 | 87.5 | 79.9 |
| Example 3-4 | 30 | 90.2 | 83.4 | 79.7 |
| Example 3-6 | 50 | 92.7 | 81.8 | 73 |
| Example 3-7 | 75 | 92.5 | 91.8 | 83 |
| Example 3-8 | 100 | 81.3 | 69.8 | 79.6 |

These studies showed that the lyophilization of mRNA-LNP formulations can be acceptable when the amount of sodium lactate is 5-100 mM; when the amount of the sodium lactate is in the range of 5-75 mM, a lyophilized formulation with optimal integrity after lyophilization can be obtained; when the amount of sodium lactate is 5-50 mM, a lyophilized formulation with excellent critical quality attributes after lyophilization can be obtained; when the amount of sodium lactate is in the range of 10-100 mM, the lyophilized formulation has excellent thermal stability. In summary, most preferably, when the concentration of sodium lactate is in the range of 10-50 mM, a lyophilized formulation with excellent critical quality attributes and excellent thermal stability after lyophilization can be obtained.

### Example 4. Exploration on amount of different buffers in combination with sodium lactate

In the studies of Example 2 and Example 3, it was found that when sodium lactate is used alone, the pH is susceptible to great fluctuations due to its weak buffering capacity, which may potentially affect the technical effect of sodium lactate on the stability of mRNA formulations during lyophilization. In order to achieve better technical effects, the target amount of sodium lactate and various buffer pair stock solutions were added to the mRNA-LNP stock solution to further explore the lyophilization effect of combinations of sodium lactate and buffer pairs of different concentrations and identities according to the specific procedures in Example 1. The specific exploration content is shown in Table 10.

As can be seen from the results in Table 11, after the addition of the buffer pair/sodium lactate combinations, lyophilization, and reconstitution, the measured pH value was very close to the target set pH value, and the comparison with Examples 2 and 3 demonstrated that the addition of the buffer pairs can significantly stabilize the pH value of the formulations. Sodium lactate was used at 20 mM in combination with different amounts of acetate, phosphate, Tris, citrate, or histidine buffer pair for lyophilization. When the amount of the buffer pair was 30 mM and 50 mM (i.e., the molar ratio of buffer salt to sodium lactate was 1.5 and 2.5), the encapsulation efficiency after lyophilization was significantly lower and the corresponding integrity was also reduced (an encapsulation efficiency of less than 80% or and integrity of less than 85%), indicating that a high molar ratio of buffer salt to sodium lactate was disadvantageous to the mRNA-LNP lyophilization. When the molar ratio of the acetate, phosphate, Tris, citrate, or histidine buffer pair to sodium lactate is in the range of 0.25-1, the combination of the buffer pair and the lactate can provide better lyophilization effects. Thus, a combination with a molar ratio of the acetate, phosphate, Tris, citrate, or histidine buffer pair to sodium lactate in the range of 0.25-1 is preferred.

**Table 10. Exploration on amount of different buffers in combination with sodium lactate**

| Sample name | Amount of sodium lactate (mM) | Acetate buffer pair (mM) | Phosphate buffer pair (mM) | Tris buffer pair (mM) | Citrate buffer pair (mM) | Histidine buffer pair (mM) | Set pH value | Theoretical mass fraction (%) of buffering salt in lyophilized solid¹ | Theoretical mass fraction (%) of sodium lactate in lyophilized solid² | Molar ratio of buffer salt to sodium lactate in lyophilized formulation³ |
|---|---|---|---|---|---|---|---|---|---|---|
| Example 4-1 | 20 | 5 | / | / | / | / | 7.5 | 0.45 | 1.47 | 0.25 |
| Example 4-2 | 20 | 10 | / | / | / | / | 7.5 | 0.9 | 1.46 | 0.5 |
| Example 4-3 | 20 | 15 | / | / | / | / | 7.5 | 1.34 | 1.46 | 0.75 |
| Example 4-4 | 20 | 20 | / | / | / | / | 7.5 | 1.78 | 1.45 | 1 |
| Example 4-5 | 20 | 30 | / | / | / | / | 7.5 | 2.65 | 1.45 | 1.5 |
| Example 4-6 | 20 | 50 | / | / | / | / | 7.5 | 4.34 | 1.43 | 2.5 |
| Example 4-7 | 20 | / | 5 | / | / | / | 7.5 | 1.18 | 1.46 | 0.25 |
| Example 4-8 | 20 | / | 10 | / | / | / | 7.5 | 2.33 | 1.45 | 0.5 |
| Example 4-9 | 20 | / | 15 | / | / | / | 7.5 | 3.46 | 1.44 | 0.75 |
| Example 4-10 | 20 | / | 20 | / | / | / | 7.5 | 4.56 | 1.43 | 1 |
| Example 4-11 | 20 | / | 30 | / | / | / | 7.5 | 6.68 | 1.41 | 1.5 |
| Example 4-12 | 20 | / | 50 | / | / | / | 7.5 | 10.66 | 1.38 | 2.5 |
| Example 4-13 | 20 | / | / | 5 | / | / | 7.5 | 0.52 | 1.47 | 0.25 |
| Example 4-14 | 20 | / | / | 10 | / | / | 7.5 | 1.03 | 1.46 | 0.5 |
| Example 4-15 | 20 | / | / | 15 | / | / | 7.5 | 1.54 | 1.46 | 0.75 |
| Example 4-16 | 20 | / | / | 20 | / | / | 7.5 | 2.04 | 1.45 | 1 |
| Example 4-17 | 20 | / | / | 30 | / | / | 7.5 | 3.03 | 1.44 | 1.5 |
| Example 4-18 | 20 | / | / | 50 | / | / | 7.5 | 4.94 | 1.43 | 2.5 |
| Example 4-19 | 20 | / | / | / | 5 | / | 7.5 | 0.97 | 1.46 | 0.25 |
| Example 4-20 | 20 | / | / | / | 10 | / | 7.5 | 1.92 | 1.45 | 0.5 |
| Example 4-21 | 20 | / | / | / | 15 | / | 7.5 | 2.86 | 1.44 | 0.75 |
| Example 4-22 | 20 | / | / | / | 20 | / | 7.5 | 3.77 | 1.44 | 1 |
| Example 4-23 | 20 | / | / | / | 30 | / | 7.5 | 5.55 | 1.42 | 1.5 |
| Example 4-24 | 20 | / | / | / | 50 | / | 7.5 | 8.93 | 1.39 | 2.5 |
| Example 4-25 | 20 | / | / | / | / | 5 | 7.5 | 0.52 | 1.47 | 0.25 |
| Example 4-26 | 20 | / | / | / | / | 10 | 7.5 | 1.03 | 1.47 | 0.5 |
| Example 4-27 | 20 | / | / | / | / | 15 | 7.5 | 1.52 | 1.46 | 0.75 |
| Example 4-28 | 20 | / | / | / | / | 20 | 7.5 | 2.04 | 1.46 | 1 |
| Example 4-29 | 20 | / | / | / | / | 30 | 7.5 | 3.05 | 1.46 | 1.5 |
| Example 4-30 | 20 | / | / | / | / | 50 | 7.5 | 5.03 | 1.45 | 2.5 |

| | | | | | | | | | | |
|---|---|---|---|---|---|---|---|---|---|---|
| ¹ The theoretical calculation was on a dry basis where water was completely removed after lyophilization. The solid matters after lyophilization were the lyoprotectant, the mRNA-LNP, sodium lactate, and the buffer pair, and the theoretical mass fraction (%) of the buffer pair in the lyophilized solid was based on the sum of the masses of the lyoprotectant, the mRNA-LNP, sodium lactate, and the buffer pair. ² The theoretical calculation was on a dry basis where water was completely removed after lyophilization. The solid matters after lyophilization were the lyoprotectant, the mRNA-LNP, sodium lactate, and the buffer pair, and the theoretical mass fraction (%) of sodium lactate in the lyophilized solid was based on the sum of the masses of the lyoprotectant, the mRNA-LNP, sodium lactate, and the buffer pair. ³ The ratio of the molar concentration of the buffer salt to the molar concentration of the sodium lactate is the molar ratio of the two in the lyophilized formulation. | | | | | | | | | | |

**Table 11. Results of exploration on amount of different buffers in combination with sodium lactate**

| Sample name | Encapsulation efficiency (%) | Integrity (%) | Particle size (nm) | Measured pH |
|---|---|---|---|---|
| mRNA-LNP stock solution before lyophilization | 96.4 | 92.1 | 85.3 | / |
| Example 4-1 | 93.3 | 89.3 | 95.4 | 7.47 |
| Example 4-2 | 89.5 | 92.0 | 99.2 | 7.55 |
| Example 4-3 | 88.4 | 93.0 | 92.3 | 7.52 |
| Example 4-4 | 91.2 | 91.7 | 97.5 | 7.50 |
| Example 4-5 | 83.1 | 83.3 | 93.1 | 7.44 |
| Example 4-6 | 79.0 | 84.0 | 98.9 | 7.49 |
| Example 4-7 | 92.1 | 93.3 | 99.2 | 7.51 |
| Example 4-8 | 90.7 | 89.7 | 98.7 | 7.55 |
| Example 4-9 | 92.3 | 92.5 | 96.3 | 7.46 |
| Example 4-10 | 91.8 | 95.6 | 95.5 | 7.49 |
| Example 4-11 | 82.3 | 83.3 | 93.4 | 7.42 |
| Example 4-12 | 78.7 | 81.2 | 98.2 | 7.58 |
| Example 4-13 | 91.4 | 88.9 | 90.7 | 7.47 |
| Example 4-14 | 90.3 | 93.7 | 91.2 | 7.63 |
| Example 4-15 | 89.2 | 91.6 | 93.5 | 7.42 |
| Example 4-16 | 91.0 | 94.2 | 94.2 | 7.49 |
| Example 4-17 | 84.2 | 84.5 | 89.7 | 7.51 |
| Example 4-18 | 81.3 | 83.2 | 93.3 | 7.48 |
| Example 4-19 | 90.3 | 89.1 | 94.4 | 7.50 |
| Example 4-20 | 91.0 | 90.1 | 92.4 | 7.58 |
| Example 4-21 | 88.6 | 90.3 | 88.2 | 7.45 |
| Example 4-22 | 89.4 | 87.9 | 90.8 | 7.49 |
| Example 4-23 | 86.4 | 81.3 | 95.5 | 7.50 |
| Example 4-24 | 85.3 | 80.6 | 98.0 | 7.47 |
| Example 4-25 | 91.5 | 92.1 | 96.1 | 7.55 |
| Example 4-26 | 90.7 | 88.2 | 95.6 | 7.51 |
| Example 4-27 | 89.7 | 90.6 | 94.3 | 7.43 |
| Example 4-28 | 88.9 | 90.1 | 90.3 | 7.61 |
| Example 4-29 | 86.3 | 75.4 | 95.6 | 7.41 |
| Example 4-30 | 84.2 | 78.9 | 92.7 | 7.37 |

As shown in Table 11, the results of the study on the combination of sodium lactate at 20 mM with different buffer salts at 5-50 mM confirmed that a buffer salt/sodium lactate molar ratio of 0.25-1 is preferred. To further verify the feasibility of the lyophilization with a buffer salt/sodium lactate molar ratio of 0.25-1, the lyophilization effects of combinations of sodium lactate at 30/40/50 mM with different buffer salts were further explored. The specific exploration content is shown in Table 12.

Combinations of sodium lactate at 30/40/50 mM with Tris buffer or histidine buffer in different molar ratios were prepared and lyophilized. The specific exploration results are shown in Table 13. When the molar ratio of the buffer salt to sodium lactate was 0.2, the pH after lyophilization and reconstitution deviated from the target pH 7.5, and when the molar ratio of buffer salt to sodium lactate was 0.25 or greater, the pH after lyophilization and reconstitution was very close to the target pH 7.5, suggesting that the pH of the formulations cannot be effectively stabilized by a combination with a low buffer salt/sodium lactate molar ratio. In a buffer salt/sodium lactate molar ratio of 1.5, the encapsulation efficiency and integrity after lyophilization and reconstitution were relatively low, despite that differences were observed at sodium lactate amount of 30/40/50 mM, indicating that an excessive amount of buffer salt may affect the effect of lyophilization. When the buffer salt/sodium lactate molar ratio is 0.25-1, the lyophilization effect is similar to that in Table 11, and the quality attributes of the formulations after lyophilization are all relatively close to those before lyophilization.

In summary, when combinations of different amounts of sodium lactate with different buffers are used for lyophilization, the pH-stabilizing effect and the lyophilization effect are better when the molar ratio of the buffer salt to sodium lactate is 0.25-1. Thus, a buffer salt/sodium lactate molar ratio of 0.25-1 is preferred for lyophilization.

**Table 12. Exploration on combinations of different buffers and different amounts of sodium lactate**

| Sample name | Amount of sodium lactate (mM) | Tris buffer pair (mM) | Histidine buffer pair (mM) | Set pH value | Theoretical mass fraction (%) of buffering salt in lyophilized solid¹ | Theoretical mass fraction (%) of sodium lactate in lyophilized solid² | Molar ratio of buffer salt to sodium lactate in lyophilized formulation³ |
|---|---|---|---|---|---|---|---|
| Example 4-31 | 30 | 6 | / | 7.5 | 0.61 | 2.18 | 0.2 |
| Example 4-32 | 30 | 7.5 | / | 7.5 | 0.77 | 2.17 | 0.25 |
| Example 4-33 | 30 | 15 | / | 7.5 | 1.52 | 2.16 | 0.5 |
| Example 4-34 | 30 | 22.5 | / | 7.5 | 2.27 | 2.14 | 0.75 |
| Example 4-35 | 30 | 30 | / | 7.5 | 3.00 | 2.13 | 1 |
| Example 4-36 | 30 | 45 | / | 7.5 | 4.43 | 2.09 | 1.5 |
| Example 4-37 | 40 | 8 | / | 7.5 | 0.81 | 2.88 | 0.2 |
| Example 4-38 | 40 | 10 | / | 7.5 | 1.01 | 2.87 | 0.25 |
| Example 4-39 | 40 | 20 | / | 7.5 | 2.00 | 2.84 | 0.5 |
| Example 4-40 | 40 | 30 | / | 7.5 | 2.98 | 2.81 | 0.75 |
| Example 4-41 | 40 | 40 | / | 7.5 | 3.93 | 2.79 | 1 |
| Example 4-42 | 40 | 60 | / | 7.5 | 5.78 | 2.73 | 1.5 |
| Example 4-43 | 50 | 10 | / | 7.5 | 1.01 | 3.56 | 0.2 |
| Example 4-44 | 50 | 12.5 | / | 7.5 | 1.25 | 3.55 | 0.25 |
| Example 4-45 | 50 | 25 | / | 7.5 | 2.48 | 3.51 | 0.5 |
| Example 4-46 | 50 | 37.5 | / | 7.5 | 3.67 | 3.47 | 0.75 |
| Example 4-47 | 50 | 50 | / | 7.5 | 4.83 | 3.43 | 1 |
| Example 4-48 | 50 | 75 | / | 7.5 | 7.08 | 3.34 | 1.5 |
| Example 4-49 | 30 | / | 6 | 7.5 | 0.60 | 2.18 | 0.2 |
| Example 4-50 | 30 | / | 7.5 | 7.5 | 0.76 | 2.18 | 0.25 |
| Example 4-51 | 30 | / | 15 | 7.5 | 1.50 | 2.17 | 0.5 |
| Example 4-52 | 30 | / | 22.5 | 7.5 | 2.25 | 2.17 | 0.75 |
| Example 4-53 | 30 | / | 30 | 7.5 | 2.99 | 2.16 | 1 |
| Example 4-54 | 30 | / | 45 | 7.5 | 4.43 | 2.14 | 1.5 |
| Example 4-55 | 40 | / | 8 | 7.5 | 0.80 | 2.89 | 0.2 |
| Example 4-56 | 40 | / | 10 | 7.5 | 0.99 | 2.89 | 0.25 |
| Example 4-57 | 40 | / | 20 | 7.5 | 1.98 | 2.87 | 0.5 |
| Example 4-58 | 40 | / | 30 | 7.5 | 2.97 | 2.86 | 0.75 |
| Example 4-59 | 40 | / | 40 | 7.5 | 3.93 | 2.84 | 1 |
| Example 4-60 | 40 | / | 60 | 7.5 | 5.83 | 2.82 | 1.5 |
| Example 4-61 | 50 | / | 10 | 7.5 | 0.99 | 3.58 | 0.2 |
| Example 4-62 | 50 | / | 12.5 | 7.5 | 1.24 | 3.58 | 0.25 |
| Example 4-63 | 50 | / | 25 | 7.5 | 2.45 | 3.56 | 0.5 |
| Example 4-64 | 50 | / | 37.5 | 7.5 | 3.67 | 3.54 | 0.75 |
| Example 4-65 | 50 | / | 50 | 7.5 | 4.84 | 3.51 | 1 |
| Example 4-66 | 50 | / | 75 | 7.5 | 7.19 | 3.47 | 1.5 |

| | | | | | | | |
|---|---|---|---|---|---|---|---|
| ¹ The theoretical calculation was on a dry basis where water was completely removed after lyophilization. The solid matters after lyophilization were the lyoprotectant, the mRNA-LNP, sodium lactate, and the buffer pair, and the theoretical mass fraction (%) of the buffer pair in the lyophilized solid was based on the sum of the masses of the lyoprotectant, the mRNA-LNP, sodium lactate, and the buffer pair. ² The theoretical calculation was on a dry basis where water was completely removed after lyophilization. The solid matters after lyophilization were the lyoprotectant, the mRNA-LNP, sodium lactate, and the buffer pair, and the theoretical mass fraction (%) of sodium lactate in the lyophilized solid was based on the sum of the masses of the lyoprotectant, the mRNA-LNP, sodium lactate, and the buffer pair. ³ The ratio of the molar concentration of the buffer salt to the molar concentration of the sodium lactate is the molar ratio of the two in the lyophilized formulation. | | | | | | | |

**Table 13. Results of exploration on combinations of different buffers and different amounts of sodium lactate**

| Sample name | Encapsulation efficiency (%) | Integrity (%) | Particle size (nm) | Measured pH |
|---|---|---|---|---|
| mRNA-LNP stock solution before lyophilization | 97.0 | 91.7 | 80.7 | / |
| Example 4-31 | 91.3 | 89.7 | 90.5 | 7.23 |
| Example 4-32 | 90.5 | 91.0 | 94.4 | 7.51 |
| Example 4-33 | 91.0 | 91.3 | 90.6 | 7.55 |
| Example 4-34 | 88.9 | 90.5 | 89.7 | 7.49 |
| Example 4-35 | 89.4 | 93.2 | 87.2 | 7.52 |
| Example 4-36 | 81.8 | 83.6 | 89.3 | 7.51 |
| Example 4-37 | 89.1 | 90.7 | 88.9 | 7.26 |
| Example 4-38 | 89.0 | 89.0 | 93.5 | 7.47 |
| Example 4-39 | 90.2 | 89.3 | 93.2 | 7.55 |
| Example 4-40 | 90.8 | 91.5 | 90.8 | 7.52 |
| Example 4-41 | 91.4 | 88.3 | 94.1 | 7.56 |
| Example 4-42 | 79.4 | 81.9 | 89.7 | 7.50 |
| Example 4-43 | 87.3 | 92.4 | 90.3 | 7.24 |
| Example 4-44 | 86.8 | 93.6 | 91.2 | 7.47 |
| Example 4-45 | 88.7 | 89.2 | 94.1 | 7.52 |
| Example 4-46 | 89.4 | 90.3 | 92.6 | 7.53 |
| Example 4-47 | 88.3 | 90.7 | 88.3 | 7.49 |
| Example 4-48 | 75.6 | 79.8 | 87.5 | 7.52 |
| Example 4-49 | 91.7 | 88.9 | 86.5 | 7.25 |
| Example 4-50 | 90.2 | 88.7 | 85.2 | 7.57 |
| Example 4-51 | 90.2 | 90.8 | 89.7 | 7.51 |
| Example 4-52 | 88.4 | 93.2 | 91.3 | 7.54 |
| Example 4-53 | 89.8 | 91.4 | 88.5 | 7.58 |
| Example 4-54 | 84.2 | 83.5 | 91.5 | 7.50 |
| Example 4-55 | 89.2 | 89.5 | 89.2 | 7.19 |
| Example 4-56 | 89.7 | 89.2 | 90.3 | 7.42 |
| Example 4-57 | 90.0 | 90.1 | 90.3 | 7.50 |
| Example 4-58 | 91.3 | 92.4 | 89.7 | 7.52 |
| Example 4-59 | 90.7 | 89.1 | 89.4 | 7.51 |
| Example 4-60 | 80.1 | 78.4 | 88.6 | 7.48 |
| Example 4-61 | 88.5 | 89.3 | 88.2 | 7.14 |
| Example 4-62 | 87.7 | 88.9 | 90.2 | 7.47 |
| Example 4-63 | 87.3 | 90.6 | 92.0 | 7.43 |
| Example 4-64 | 89.5 | 91.2 | 89.6 | 7.48 |
| Example 4-65 | 86.4 | 89.1 | 90.5 | 7.56 |
| Example 4-66 | 72.4 | 75.6 | 87.4 | 7.51 |

### Example 5. Exploration on lyophilization effect at different pH

On the basis of Examples 1-4, the optimum pH range of lyophilized formulations containing sodium lactate before lyophilization was further explored. On the basis that the amount of sodium lactate was 20 mM, the lyophilization effects of sodium lactate alone or in combination with different buffer pairs at 10 mM at different pH values were explored. Different buffer pairs were combined with sodium lactate and adjusted to the target pH with a suitable pH regulator. The specific lyophilization results are shown in Table 14.

**Table 14. Lyophilization effect at the same pH**

| Sample name | Amount of sodium lactate (mM) | Buffer pair (10 mM) | Set pH | Encapsulation efficiency (%) | Integrity (%) | Particle size (nm) | pH after lyophilization |
|---|---|---|---|---|---|---|---|
| mRNA-LNP stock solution before lyophilization | / | / | / | 95.9 | 93.5 | 84.2 | / |
| Example 5-1 | 20 | Acetate buffer pair | 6.5 | 83.3 | 89.3 | 115.4 | 6.47 |
| Example 5-2 | 20 | Phosphate buffer pair | 6.5 | 79.5 | 92.0 | 119.2 | 6.55 |
| Example 5-3 | 20 | Tris buffer pair | 6.5 | 78.4 | 93.0 | 112.3 | 6.52 |
| Example 5-4 | 20 | Citrate buffer pair | 6.5 | 81.2 | 91.7 | 117.5 | 6.50 |
| Example 5-5 | 20 | Histidine buffer pair | 6.5 | 83.1 | 90.3 | 123.1 | 6.44 |
| Example 5-6 | 20 | Acetate buffer pair | 7.0 | 90.1 | 93.3 | 99.2 | 7.01 |
| Example 5-7 | 20 | Phosphate buffer pair | 7.0 | 90.7 | 89.7 | 98.7 | 7.05 |
| Example 5-8 | 20 | Tris buffer pair | 7.0 | 91.3 | 92.5 | 96.3 | 6.96 |
| Example 5-9 | 20 | Citrate buffer pair | 7.0 | 91.2 | 95.6 | 95.5 | 6.99 |
| Example 5-10 | 20 | Histidine buffer pair | 7.0 | 88.3 | 93.3 | 93.4 | 6.92 |
| Example 5-11 | 20 | Acetate buffer pair | 7.5 | 93.4 | 88.9 | 90.7 | 7.47 |
| Example 5-12 | 20 | Phosphate buffer pair | 7.5 | 92.3 | 93.7 | 91.2 | 7.63 |
| Example 5-13 | 20 | Tris buffer pair | 7.5 | 89.2 | 91.6 | 93.5 | 7.42 |
| Example 5-14 | 20 | Citrate buffer pair | 7.5 | 93.0 | 94.2 | 94.2 | 7.49 |
| Example 5-15 | 20 | Histidine buffer pair | 7.5 | 92.2 | 87.5 | 89.7 | 7.51 |
| Example 5-16 | 20 | Acetate buffer pair | 8.0 | 91.3 | 84.1 | 94.4 | 8.00 |
| Example 5-17 | 20 | Phosphate buffer pair | 8.0 | 90.0 | 85.1 | 92.4 | 8.08 |
| Example 5-18 | 20 | Tris buffer pair | 8.0 | 89.6 | 85.3 | 88.2 | 7.95 |
| Example 5-19 | 20 | Citrate buffer pair | 8.0 | 89.5 | 82.9 | 90.8 | 7.99 |
| Example 5-20 | 20 | Histidine buffer pair | 8.0 | 88.4 | 81.3 | 95.5 | 8.00 |
| Example 5-21 | 20 | Acetate buffer pair | 8.5 | 91.5 | 77.1 | 96.0 | 8.55 |
| Example 5-22 | 20 | Phosphate buffer pair | 8.5 | 92.7 | 73.2 | 94.2 | 8.51 |
| Example 5-23 | 20 | Tris buffer pair | 8.5 | 90.7 | 75.6 | 93.5 | 8.43 |
| Example 5-24 | 20 | Citrate buffer pair | 8.5 | 88.7 | 70.1 | 90.3 | 8.61 |
| Example 5-25 | 20 | Histidine buffer pair | 8.5 | 89.3 | 65.4 | 95.6 | 8.41 |

As can be seen from Table 14, at pH 6.5, the increases in particle size after lyophilization in formulations containing sodium lactate and different buffer pairs were significantly greater than those of other pH values; the particle size after lyophilization and reconstitution was greater than 110 nm, and the encapsulation efficiency after lyophilization and reconstitution was lower than those of other pH values. At pH 8.5, the integrity after lyophilization was significantly reduced as compared to that before lyophilization. At pH 7.0-8.0, formulations containing sodium lactate and different buffer pairs were well lyophilized with the quality attributes after lyophilization close to those before lyophilization. When 20 mM sodium lactate was used alone for lyophilization, a good effect was observed at pH 7.27 (between 7 and 8).

In summary, when an mRNA-LNP sample containing the lactate is lyophilized, regardless of whether sodium lactate is used alone or in combination with different buffer pairs, a lyophilization effect can be obtained within a pH range of 7-8. Thus the preferred lyophilization pH range is 7-8.

### Example 6. Lyophilization effect of different types of mRNA

Based on the exploration on conventional linear mRNA, it was further confirmed whether the lyophilization conditions available on the conventional linear mRNA are suitable for the self-amplifying mRNA (samRNA) and the circular mRNA (circRNA). For example, the samRNA and the circRNA of eGFP were encapsulated in the same manner as in the above examples, the exchange buffer after encapsulation was 50 mM of sodium lactate & 10 mM of PB buffer containing a lyoprotectant. The mixture was aliquoted into 2R vials at 0.6 mL per vial and then lyophilized as per the process in Example 1. The lyophilization results are shown in Table 15. The samples in Table 15 were lyophilized and reconstituted with enzyme-free water, and were taken for expression assay in cells. Specifically, Hep3B cells with a cell density of 6.5 × 10⁵ cells/mL were seeded on a 6-well plate at 1 mL/well. After 24 hours of incubation, 2.5 µg of mRNA-LNP was added to each well for transfection, and the plate was incubated in a 37 °C, 5% CO₂ cell incubator. The negative control group was transfected with physiological saline with the same volume. After 24 hours, the microscopic images were taken. The results are shown in FIG. 8.

**Table 15. Lyophilization effect of different types of mRNA**

| Sample name | | Type of mRNA | Particle size (nm) | PDI | Encapsulation efficiency (%) | Integrity (%) |
|---|---|---|---|---|---|---|
| Example 6-1 | Before lyophilization | SamRNA (eGFP) | 78.9 | 0.06 | 99.2 | 83.5 |
| | After lyophilization | | 85.2 | 0.05 | 95.6 | 81.2 |
| Example 6-2 | Before lyophilization | circRNA (eGFP) | 83.5 | 0.09 | 97 | 92.3 |
| | After lyophilization | | 88.2 | 0.11 | 92.4 | 93.1 |
| Comparative Example 6-1 | Before lyophilization | Conventional linear mRNA (eGFP) | 81.5 | 0.07 | 95.7 | 95.4 |
| | After lyophilization | | 90.3 | 0.07 | 91.2 | 94.2 |

As can be seen from Table 15, the three different mRNA molecules were well encapsulated and lyophilized, and various attributes after lyophilization were very close to those before lyophilization. As can be seen in FIG. 8, the cell expression levels before and after lyophilization of the three types of mRNA were very close, indicating that the lyophilization did not result in a decrease in the biological activity of the 3 types of mRNA formulations. The results suggest that the formulations and process for lyophilization have high compatibility with different types of nucleic acids, and the different types of nucleic acid can be well lyophilized.

Samples of Examples 6-1 and 6-2 were SamRNA and circRNA. To further demonstrate the performance of the lactate salt in lyophilization, the samples of Examples 6-1 and 6-2 were subjected to electron microscopy.

On a graphene carrier net, the sample was placed on a watch glass with the front side facing upwards, and detected on a glow discharge spectrometer for 2 seconds (the detection was manually stopped). The lyophilized samples were prepared, 1) the lyophilizer was started half an hour in advance, the filter paper was replaced, double distilled water was added into the lyophilizer, and the lyophilization parameters of Blot Time (2.5 s), Blot Force (3 s), Wait Time (3 s) and Blot Total (1 s) were input for sample preparation.

Liquid nitrogen and liquid ethane were added into a mold. The carrier net was clamped on a clamp according to the direction of the clamping groove, and then the clamp and the carrier net were mounted on the lyophilizer; 3 µL of the reconstituted samples of Examples 6-1 and 6-2 were carefully transferred to the surface of the carrier net by a pipette (care was taken to prevent the pipette tip from puncturing the carrier net), and the instrument was started according to the sample preparation conditions; the clamp was removed, and the carrier net was quickly transferred from ethane to liquid nitrogen (care was taken to prevent net from bending) and mounted in an electron microscopic rotating wheel die. After the samples were preserved, the lyophilization was completed.

The frozen carrier net was loaded on an FEI 200KV Talos Arctica G2 field emission transmission electron microscope, and the sample was cooled and loaded for electron microscopy and photography.

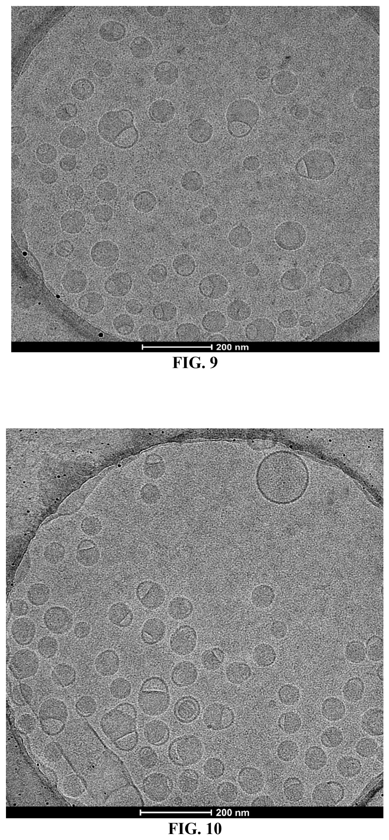

As can be seen from the electron microscopic images of the samples of Examples 6-1 (FIG. 9) and 6-2 (FIG. 10), even at relatively high salt concentrations of 50 mM for sodium lactate and 10 mM for PB buffer, the LNPs encapsulating SamRNA or circRNA still exhibited uniform spherical shapes after lyophilization and reconstitution, suggesting that the micro-morphology of the formulation was not damaged by the lyophilization.

### Example 7. Lyophilization study on different lipid nanoparticles

Taking eGFP mRNA as an example, the LNP-mRNA encapsulation was performed with different cationic lipids. Three cationic lipids were dissolved in ethanol in molar ratios in Table 16, and mRNA was dissolved in acetate buffer pH 4.0. An mRNA-LNP composition was prepared using a microfluidic device (nanoparticle preparation apparatus Ignite from Precision Nanosystems) with a lipid mixture:mRNA flow rate ratio of 1:3 (see Table 16 for the formulation). The encapsulated mRNA-LNP was transferred to the buffers in Table 16, and the sodium lactate stock solution was added at the target concentration. The lyoprotectant stock solution was added to make the final lyoprotectant composition identical to that in Example 1. The mixture was subjected to sterile filtration and aliquoted into 2R vials at 0.6 mL per vial for lyophilization. Samples were collected before and after lyophilization to determine encapsulation efficiency, average particle size, PDI, and integrity, as well as expression assay in cells.

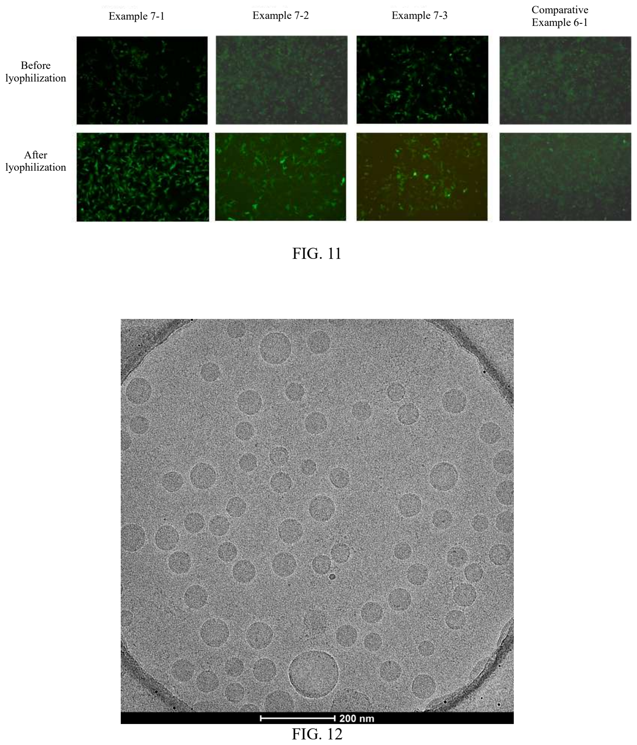

The attributes of LNPs before and after encapsulation and lyophilization are shown in Table 17. mRNA were encapsulated well by the three different lipid nanoparticles in different nitrogen-phosphorus ratios, and the attributes of the prepared LNPs were all excellent. The samples were lyophilized after combinations of sodium lactate at different amounts and different buffers were prepared. The physicochemical attributes after lyophilization were very close to those before lyophilization, suggesting good lyophilization of the samples. The expression results in cells before and after lyophilization are shown in FIG. 11, and the expressions of the three LNP formulations in cells after lyophilization were relatively high and even higher than those of the three LNP formulations before lyophilization.

**Table 16. Different three-component LNP formulations**

| Sample name | Amount of sodium lactate (mM) | Buffer composition | Lipid formulation (molar proportion) | Nitrogen-to-phosphorus ratio |
|---|---|---|---|---|
| Example 7-1 | 20 | 10mM PB | ALC-0315:cholesterol:DSPC:DMG-PEG2000 = 49:10:39.5:1.5 | 6 |
| Example 7-2 | 30 | 15 mM histidine | MC3:cholesterol:DSPC:DMG-PEG2000 = 49:10:39.5:1.5 | 4 |
| Example 7-3 | 50 | 25 mM citrate | SM-102:cholesterol:DSPC:DMG-PEG2000 = 49:10:39.5:1.5 | 8 |

**Table 17. Lyophilization effect of three-component LNPs**

| Sample name | | Cationic lipid | Particle size (nm) | PDI | Encapsulation efficiency (%) | Integrity (%) |
|---|---|---|---|---|---|---|
| Example 7-1 | Before lyophilization | ALC-0315 | 75.4 | 0.08 | 98.2 | 93.2 |
| | After lyophilization | | 82.6 | 0.09 | 95.0 | 89.5 |
| Example 7-2 | Before lyophilization | MC3 | 81.7 | 0.10 | 97 | 94.3 |
| | After lyophilization | | 87.6 | 0.08 | 93.4 | 92.3 |
| Example 7-3 | Before lyophilization | SM-102 | 80.2 | 0.11 | 99.7 | 94.4 |
| | After lyophilization | | 90.2 | 0.10 | 95.2 | 96.2 |

On a graphene carrier net, the sample was placed on a watch glass with the front side facing upwards, and detected on a glow discharge spectrometer for 2 seconds (the detection was manually stopped). The lyophilized samples were prepared, 1) the lyophilizer was started half an hour in advance, the filter paper was replaced, double distilled water was added into the lyophilizer, and the lyophilization parameters of Blot Time (2.5 s), Blot Force (3 s), Wait Time (3 s) and Blot Total (1 s) were input for sample preparation.

Liquid nitrogen and liquid ethane were added into a mold. The carrier net was clamped on a clamp according to the direction of the clamping groove, and then the clamp and the carrier net were mounted on the lyophilizer; 3 µL of the reconstituted samples of Examples 7-1, 7-2, and 7-3 were carefully transferred to the surface of the carrier net by a pipette (care was taken to prevent the pipette tip from puncturing the carrier net), and the instrument was started according to the sample preparation conditions; the clamp was removed, and the carrier net was quickly transferred from ethane to liquid nitrogen (care was taken to prevent net from bending) and mounted in an electron microscopic rotating wheel die. After the samples were preserved, the lyophilization was completed.

The frozen carrier net was loaded on an FEI 200KV Talos Arctica G2 field emission transmission electron microscope, and the sample was cooled and loaded for electron microscopy and photography.

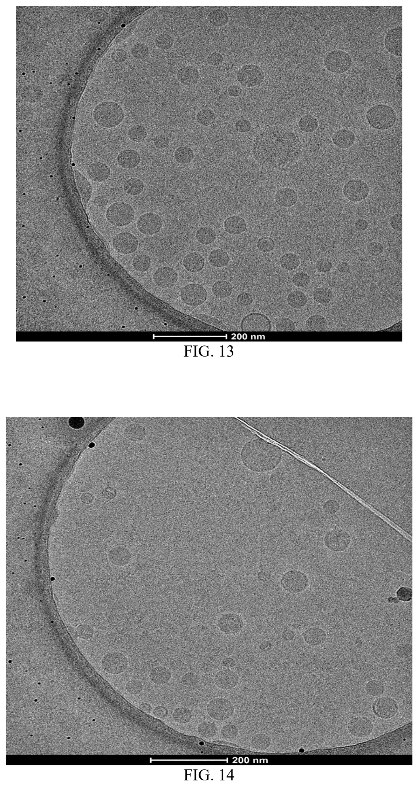

As can be seen from the electron microscopic images of the samples of Examples 7-1 (FIG. 12), 7-2 (FIG. 13), and 7-3 (FIG. 14), the three LNP formulations exhibited micro-morphology with no defects after lyophilization.

Finally, it should be noted that, the above examples are only used to illustrate the embodiments of the present disclosure, and should not limit the same. Although the present disclosure is described in detail with reference to the examples described above, it will be appreciated by those skilled in the art that, the embodiments in the examples described above can still be modified, or some or all of the technical features can be equivalently substituted. Such modifications or substitutions do not make the embodiments corresponding thereto depart from the scope of the embodiments in the examples of the present invention.

## Claims

1. A nucleic acid-lipid nanoparticle composition, comprising: a nucleic acid, a lipid, and a lactate, wherein the lactate is selected from sodium lactate or potassium lactate in an amount of 5-100 mM, for example, 5-75 mM, 5-50 mM, or 10-100 mM, preferably 5-50 mM.

2. The nucleic acid-lipid nanoparticle composition according to claim 1, further comprising a buffer, wherein the content ratio of the buffer to the lactate is (0.25-1):1.

3. The nucleic acid-lipid nanoparticle composition according to claim 2, wherein the buffer is one selected from or a combination of more selected from the group consisting of an acetate buffer, a PBS buffer, a citrate buffer, a phosphate buffer, a histidine buffer, and a tris buffer; preferably, the content of the buffer is 1.25-50 mM.

4. The nucleic acid-lipid nanoparticle composition according to any one of claims 1-3, further comprising a lyoprotectant, wherein preferably, the lyoprotectant is one selected from or a combination of more selected from the group consisting of sucrose, trehalose, lactose, cyclodextrin, and mannitol.

5. The nucleic acid-lipid nanoparticle composition according to any one of claims 1-4, wherein the pH of the nucleic acid-lipid nanoparticle composition is 1.0-14.0, for example, 2.0-13.0, preferably 5.0-9.0, and more preferably 7.0-8.0.

6. The nucleic acid-lipid nanoparticle composition according to any one of claims 1-5, wherein the nucleic acid is selected from the group consisting of ssDNA, dsDNA, mRNA, lncRNA, siRNA, saRNA, shRNA, ASO, plasmid, circRNA, circDNA, miRNA, CRISPR-Cas, ployI:C, SamRNA, and 5'-pppRNA.

7. The nucleic acid-lipid nanoparticle composition according to any one of claims 1-6, wherein the nucleic acid is encapsulated in a lipid nanoparticle (LNP).

8. The nucleic acid-lipid nanoparticle composition according to any one of claims 1-7, wherein the lipid comprises a cationic lipid, a neutral phospholipid, a PEG-lipid, and optionally a steroidal lipid.

9. The nucleic acid-lipid nanoparticle composition according to any one of claims 1-8, wherein the composition is prepared into a lyophilized formulation.

10. The nucleic acid-lipid nanoparticle composition according to claim 9, wherein the content of the lactate is 0.5-10.0 wt%, for example, 0.5-7.5 wt%, 0.5-5.0 wt%, or 1.0-10 wt%, preferably 0.5-5.0 wt%, on dry basis by the weight of the lyophilized formulation.

11. The nucleic acid-lipid nanoparticle composition according to claim 9 or 10, wherein the content of the buffer is 0.35-10.0 wt%, for example, 0.35-7.5 wt%, 0.35-5.0 wt%, or 1.0-10 wt%, preferably 0.5-5.0 wt%, on dry basis by the weight of the lyophilized formulation.

12. A method for preparing a nucleic acid-lipid nanoparticle composition, wherein the composition is prepared into a lyophilized formulation, and the method comprises:
(1) mixing a lactate solution with a nucleic acid-lipid nanoparticle stock solution, or
adding a lactate to a nucleic acid solution before encapsulating the nucleic acid, and adding a lipid, or
ultrafiltering a nucleic acid-lipid nanoparticle into a solution containing lactate,
wherein the lactate is selected from sodium lactate or potassium lactate in an amount of 5-100 mM, for example, 5-75 mM, 5-50 mM, or 10-100 mM, preferably 5-50 mM;
(2) adding a lyoprotectant; and
(3) lyophilizing to give a lyophilized formulation of the nucleic acid-lipid nanoparticle composition.

13. The method according to claim 12, wherein the lyoprotectant is one selected from or a combination of more selected from the group consisting of sucrose, trehalose, lactose, cyclodextrin, and mannitol.

14. The method according to claim 12 or 13, wherein the content of the lyoprotectant is 50-99.9 wt%, on dry basis by the weight of the lyophilized formulation.

15. The method according to claim 14, wherein step (1) further comprises adding a buffer, the content ratio of the buffer to the lactate being (0.25-1):1.

16. The method according to claim 15, wherein the buffer is one selected from or a combination of more selected from the group consisting of an acetate buffer, a PBS buffer, a citrate buffer, a phosphate buffer, a histidine buffer, and a tris buffer; preferably, the content of the buffer is 1.25-50 mM.

17. The method according to any one of claims 12-16, wherein the pH of the solution prepared in step (1) is 1.0-14.0, for example, 2.0-13.0, preferably 5.0-9.0, and more preferably 7.0-8.0.

18. The method according to any one of claims 12-17, wherein the nucleic acid is selected from the group consisting of ssDNA, dsDNA, mRNA, lncRNA, siRNA, saRNA, shRNA, ASO, plasmid, circRNA, circDNA, miRNA, CRISPR-Cas, ployI:C, SamRNA, and 5'-pppRNA.

19. The method according to any one of claims 12-18, wherein in step (1), the nucleic acid is encapsulated in a lipid nanoparticle.

20. The method according to any one of claims 12-19, wherein the lipid comprises a cationic lipid, a neutral phospholipid, a PEG-lipid, and optionally a steroidal lipid.

21. Use of the nucleic acid-lipid nanoparticle composition according to any one of claims 1-11 in preparing a medicament.

22. The use according to claim 21, wherein the medicament is a vaccine.

23. The use according to claim 22, wherein the vaccine is a therapeutic tumor vaccine or a vaccine for preventing a cancer, a viral infection, a bacterial infection, or a fungal infection.

24. The use according to claim 23, wherein the virus is selected from the group consisting of norovirus, Ebola virus, coronavirus, cytomegalovirus, Dengue virus, Zika virus, enterovirus, hepatitis virus, herpes simplex virus, human papilloma virus, influenza virus, Marburg virus, measles virus, poliovirus, rabies virus, and rotavirus.

25. The use according to any one of claims 21-24, wherein the medicament is a formulation for oral administration, intramuscular injection, subcutaneous injection, intravenous injection, or inhalation.

26. The use according to claim 25, wherein the formulation for inhalation is an atomized inhalant or a dry powder inhalant.
